# EUROPÄISCHE PATENTANMELDUNG

(11) **EP 2 168 632 A2**
(43) Veröffentlichungstag der Anmeldung: **31.03.2010**
(21) Anmeldenummer: 09171285.1
(22) Anmeldetag: 24.09.2009
(51) Int. Cl.: A61N 5/06, A61H 33/06, A61M 21/00

(54) **Wärme- und/oder Infrarotkabine**

(30) Priorität: 24.09.2008 DE 202008008848 U
(71) Anmelder: Lothos Spa GmbH, 1060 Wien (AT)
(72) Erfinder: Gstöttner, Michael, A-2380, Perchtoldsdorf (AT)
(74) Vertreter: Benninger, Johannes

(57) **Zusammenfassung**

Die Erfindung betrifft eine Wärme- und/oder Infrarotkabine (1) für mindestens eine Person, mit einem Boden (24) und einer Decke (20) und sich einer zwischen dem Boden (24) und der Decke (20) erstreckenden umlaufenden Seitenwand (46), mit mindestens einem säulenartigen, länglichen und/oder drehbar gelagertes Multifunktionselement (10) und/oder -bauteil, das mit dem Boden (24) und/oder der Decke (20) und/oder der Seitenwand (46) verbunden ist, wobei das Multifunktionselement wenigstens eine, insbesondere jedoch mehrere der folgenden Elemente aufweist bzw. Funktionen erfüllt (10):
- eine statische und/oder tragende Funktion, insbesondere zur Aufhängung und/oder zur Stützung weiterer Funktionselemente;
- Anordnung wenigstens eines Wärmestrahlers am oder im Multifunktionselement;
- Anordnung wenigstens einer Ausströmstelle für Aroma- und/oder Duftstoffe;
- Luftführung, -leitung, insbesondere mittels innerhalb des Multifunktionselements (10) geführter bzw. verlaufender Kanäle und/oder Leitungen und/oder Belüftungsfunktion, insbesondere mittels innerhalb und/oder außerhalb der Kabine (1) angeordneter Ein- und Ausströmöffnungen;
- Anordnung wenigstens einer Steuerungs-, Bedienungs- und/oder Anzeigeeinheit;
- Führung von Versorgungsleitungen, bspw. für Luft, Wasser, Aromastoffe etc. innerhalb von Kanälen im Multifunktionselement;
- weitere Funktionen wie Handtuchhalter etc., insbesondere durch variable Form, variablen Verlauf und Gestaltung.

## Beschreibung

Die vorliegende Erfindung betrifft eine Wärme- und/oder Infrarotkabine für mindestens eine Person, mit einem Boden und einer Decke und sich einer zwischen dem Boden und der Decke erstreckenden umlaufenden Seitenwand, mit mindestens einem Multifunktionselement, das mit dem Boden und/oder der Decke und/oder der Seitenwand verbunden ist, und dem mindestens ein Wärmestrahler zugeordnet sein kann.

Wärmekabinen sind bekannt; sie werden beispielsweise zur Behandlung von Personen bzw. bestimmter Körperregionen durch Wärmeeinwirkung eingesetzt. Die im Inneren solcher Wärmekabinen erzeugte Infrarotstrahlung wird unter anderem zur Steigerung des Wohlbefindens, ggf. aber auch zur Linderung von Gelenkbeschwerden o. dgl. angewendet. Derartige Wärmekabinen weisen üblicherweise mehrere Infrarotstrahler auf.

Eine solche Bestrahlungsvorrichtung zur Infrarotbestrahlung geht bspw. aus der DE 31 24 335 A1 hervor. Die Bestrahlungsvorrichtung weist einen oder mehrere Strahler mit einem einseitig offenen Flachgehäuse auf, das Primärreflektoren enthält. Um zu erreichen, dass aus der Bestrahlungsvorrichtung ausschließlich eine gleichmäßige und flächenhaft diffus gestreute Strahlung durch die umgrenzte Öffnung des Gehäuses in eine gewünschte Richtung austritt, sind die Strahler mit ihren Primärreflektoren innerhalb des Gehäuses derart angeordnet und ist dieses selbst derart ausgebildet, dass die von den Strahlen-Reflektor-Einheiten ausgesandte Strahlung ausschließlich auf die Innenwandungen des Gehäuses trifft, die mit diffus reflektierendem Material ausgekleidet sind und diese gestreute Strahlung durch die Gehäuseöffnung nach außen emittieren.

Ein Gehäuse zur Aufnahme eines Strahlers zur Wärmebehandlung geht weiterhin aus der DE 20 2006 000 729 U1 hervor. Das Gehäuse umfasst einen Gehäuseboden und an diesem angeordnete Seiten- und Stirnwandungen, die gegenüber dem Gehäuseboden geneigt sind. In dem von dem Gehäuseboden und den Seiten- und Stirnwandungen umgebenen Raumvolumen ist der Strahler aufgenommen. Auf der dem Gehäuseboden gegenüberliegenden Seite des Strahlers und an den Seiten- und/oder Stirnwandungen ist ein Konterreflektor angeordnet.

In der deutschen Gebrauchsmusterschrift DE 20 2008 004 045 U1 ist eine Steuervorrichtung für Wärmekabinen, insbesondere für Infrarot-Wärmekabinen beschrieben, die den typischerweise innerhalb der Kabine auftretenden wechselnden Temperatur- und Feuchtebedingungen standhält. Die Steuervorrichtung umfasst ein sog. Bedienpaneel, in dem ein Bedienelement integriert ist. Mit diesem Bedienelement kann der Benutzer verschiedene Betriebsparameter der Wärmekabine einstellen und die Betriebsbedingungen nach seinen Wünschen und Bedürfnissen individuell einstellen und gestalten.

Ein Infrarotbestrahlungsgerät zur allgemeinen Körpererwärmung, insbesondere zur steuerbaren Erhöhung der Körpertemperatur, mit einer Anordnung von senkrecht nach unten strahlenden Infrarot-Weißglühstrahlern, die in einer einheitlichen, fahrbaren Vorrichtung verbunden sind, geht aus der DE 18 14 107 U hervor.

Eine Steuervorrichtung für eine Infrarot-Wärmekabine ist aus der AT 010 566 U1 bekannt. Die Steuervorrichtung weist einen Steuerausgang zum Steuern der Heizleistung einer IR-Strahlungsquelle einer IR-Wärmekabine auf. Eine mit dem Steuerausgang der Steuervorrichtung verbundene Messvorrichtung erfasst die aus der Richtung eines bestrahlten Hautareals eines Benutzers der IR-Wärmekabine einfallende Strahlung. Die Steuervorrichtung steuert den Steuerausgang in Abhängigkeit von der Temperatur des bestrahlten Hautareals an. Die Steuervorrichtung kann weiterhin mit einer Kompensationsvorrichtung versehen sein, durch welche die Intensität der von dem bestrahlten Hautareal reflektierten Strahlung der IR-Strahlungsquelle zumindest in ausgewählten Spektralbereichen reduzierbar ist.

Die DE 195 04 512 C2 offenbart weiterhin eine Wärmebehandlungsvorrichtung mit einer aus einem textilen Flächengebilde bestehenden, an einem Gestell befestigten Kabine, die mit einer Einstiegsöffnung versehen ist. Die Einstiegsöffnung ist mit einer Verschlusseinrichtung wie bspw. einem diagonal über eine der Seitenwände der Kabine verlaufenden Reißverschluss verschlossen, der über einen Zugstab oder eine anderweitige Betätigungseinrichtung betätigbar ist.

Herkömmliche Wärmekabinen werden meist individuell gefertigt und montiert, so dass für verschiedene Größen, Einbaukonfigurationen und Innenraumgestaltungen jeweils maßgefertigte Kabinen erforderlich sind, die oftmals nicht in einer Serienfertigung, sondern als Einzelstücke gebaut werden. Wünscht bspw. ein Kunde eine bestimmte Anordnung und Konfiguration von Wärmestrahlern und/oder der zu deren Steuerung notwendigen Bedienungsoberfläche, so gibt es hierfür oftmals kein vorbereitetes Design, so dass eine Einzelfertigung notwendig ist.

Als vorrangige Aufgabe der vorliegenden Erfindung wird es daher angesehen, eine universell konfigurierbare und zu fertigende Wärmekabine zur Wärmebehandlung von Personen zur Verfügung zu stellen, die jeweils durch geringe bzw. minimale Designvariationen an die unterschiedlichsten Gestaltungs-, Konfigurations- und Ausstattungsbedürfnisse unterschiedlicher Anwender angepasst werden kann.

Diese Aufgabe wird mit dem Gegenstand des unabhängigen Anspruchs erreicht. Merkmale vorteilhafter Weiterbildungen der Erfindung ergeben sich aus den abhängigen Ansprüchen.

Die vorliegende Erfindung beschreibt eine Wärme- und/oder Infrarotkabine für mindestens eine Person, mit einem Boden und einer Decke und sich einer zwischen dem Boden und der Decke erstreckenden umlaufenden Seitenwand. Die erfindungsgemäße Kabine weist mindestens ein säulenartiges, längliches und/oder drehbar gelagertes Multifunktionselement und/oder -bauteil auf, das mit dem Boden und/oder der Decke und/oder der Seitenwand verbunden ist. Das Multifunktionselement kann wenigstens eine, insbesondere jedoch mehrere der folgenden Elemente aufweist bzw. Funktionen erfüllen:
- eine statische und/oder tragende Funktion, insbesondere zur Aufhängung und/oder zur Stützung weiterer Funktionselemente;
- Anordnung wenigstens eines Wärmestrahlers am oder im Multifunktionselement;
- Anordnung wenigstens einer Ausströmstelle für Aroma- und/oder Duftstoffe;
- Luftführung, -leitung, insbesondere mittels innerhalb des Multifunktionselements geführter bzw. verlaufender Kanäle und/oder Leitungen und/oder Belüftungsfunktion, insbesondere mittels innerhalb und/oder außerhalb der Kabine angeordneter Ein- und Ausströmöffnungen;
- Anordnung wenigstens einer Steuerungs-, Bedienungs- und/oder Anzeigeeinheit;
- Führung von Versorgungsleitungen, bspw. für Luft, Wasser, Aromastoffe etc. innerhalb von Kanälen im Multifunktionselement;
- weitere Funktionen wie Handtuchhalter etc., insbesondere durch variable Form, variablen Verlauf und Gestaltung.

Das Multifunktionselement kann als ein statisches und/oder tragendes und/oder konstruktives und/oder funktionales Bauteil der Wärme- bzw. Infrarotkabine und/oder als Rohr mit wenigstens zwei Kammern und/oder mit Kabelführungen ausgebildet sein. Zudem kann dem Multifunktionselement und/oder der Wärmekabine mindestens ein Infrarot- und/oder Wärmestrahler zugeordnet ist, der integraler Bestandteil des Multifunktionselementes oder an diesem befestigt ist und/oder der an der Seitenwand angeordnet ist. Dieser wenigstens eine am Multifunktionselement angeordnete oder diesem zugeordnete Wärmestrahler kann horizontal nach allen Seiten schwenkbar und/oder vertikal neigbar und/oder entlang einer Längserstreckungsrichtung des Multifunktionselements verschiebbar ausgebildet sein. Wahlweise kann zudem oder alternativ das Multifunktionselement selbst drehbar gelagert sein.

Eine weitere Variante der Kabine kann vorsehen, dass das Multifunktionselement wichtige Luftführungs- und/oder Belüftungsaufgaben erfüllt. So können die Öffnungsquerschnitte von Zu- und/oder Abluftöffnungen am/im Multifunktionselement variabel steuerbar und/oder verschließbar sein. Die Strömungsquerschnitte können variabel bzw. an die erforderliche Luftführung angepasst sein. Es können mehrere Öffnungen unterschiedlichen Querschnitts und/oder variablen Querschnitts in unterschiedlichen Höhen bzw. Positionen vorgesehen sein. Eine statische Belüftung kann mittels eines Kamineffekts erfolgen. Eine sog. mechanische Belüftung kann mit Hilfe eines oder mehrerer Ventilator(s) erfolgen bzw. unterstützt werden. Innerhalb der Luftführung können ggf. Luftfilter vorgesehen sein, bspw. um die Belüftung für Allergiker angenehmer zu machen. Die Luft kann auch bspw. durch Kohlefilter geleitet und/oder ggf. auch durch ein Aromasystem geleitet werden. Wahlweise können auch in einer der Seitenwände und/oder der Decke der Wärmekabine und/oder am bzw. im Multifunktionselement wenigstens ein Aromasystem und/oder ein Duftzerstäuber angeordnet sein.

Weiterhin kann eine Bedien- und/oder Anzeigeeinheit fest oder lösbar mit dem Multifunktionselement verbunden sein, über die dem Multifunktionselement und/oder der Wärmekabine zugeordnete Funktionen - z.B. die Steuerung von Leuchteinheiten, deren An- oder Abschaltung - und/oder Parameter einstellbar und/oder veränderbar sind. Weiterhin können dem Multifunktionselement Einrichtungen zur Akustikwiedergabe, insbesondere zur Medienwiedergabe, zur akustischen Übertragung von Ansagen, von Musik, von Hinweisen etc. und/oder zur visuellen Wiedergabe, insbesondere von Videos o. dgl. zugeordnet sein. Auch kann das Multifunktionselement als Leuchtkörper ausgebildet sein und flächige und/oder punktuelle Lichtaustrittsstellen aufweisen.

Besonders vorteilhaft ist es, wenn die dem Multifunktionselement zugeordnete oder an diesem angeordnete Bedieneinheit mit einer Steuerungseinheit verbunden ist, die mit jedem einzelnen Wärmestrahler in der Wärmekabine kommunikativ verbunden ist, so dass die Wärmeleistung jedes einzelnen Infrarot- oder Wärmestrahlers selektiv regelbar ist. Zumindest einer der Strahler, insbesondere jedoch alle Strahler können eine kaminartige Hinterlüftung zur Kühlung des Strahlers aufweisen. Auch kann das Multifunktionselement wahlweise einen passiven Verbrennungsschutz aufweisen, insbesondere in Form einer Oberflächenbeschichtung. Darüber hinaus kann ein wärmeisolierter Griff vorhanden sein, der zum Schwenken, Schieben, Drehen, Wenden etc. des wenigstens einen Strahlers und/oder des Multifunktionselements dienen kann. Alternativ oder zusätzlich kann das Multifunktionselement einen aktiven Verbrennungsschutz aufweisen, insbesondere in Form einer Sensoreinrichtung zur Messung der Hauttemperatur eines Benutzers und einer damit gekoppelten selektiven Steuerung der einzelnen Strahler, wobei eine Sitzbelegungserkennung bei nicht vom Sensor erfassbarer Hauttemperatur vorzugsweise den zugehörigen Strahler deaktiviert. So kann z.B. ein Sensor an jedem Strahler vorgesehen sein, der mit einer Regelung verbunden ist, in Abhängigkeit vom Sensorwert den/die Strahler aktiviert und deaktiviert bzw. in ihrer Intensität steuert. Eine solche Belegungserkennung bietet den Vorteil der Abschaltung der Strahler bei nicht benutzer Kabine und/oder nicht belegtem Sitzplatz.

Weiterhin kann wenigstens ein Sitzbereich eine ergonomisch geformte Rückenlehne aufweisen, wobei die Rückenlehne wenigstens zwei im Wesentlichen vertikal verlaufende, beabstandet voneinander angeordnete Lehnenabschnitte aufweist und/oder bei der der Rückenlehne wenigstens ein Wärmestrahler zugeordnet ist, dessen Abstrahlbereich auf den Rücken der den Sitzbereich benutzenden Person gerichtet ist, und/oder bei der die Rückenlehne und/oder die wenigstens zwei Lehnenabschnitte in mehrere, vertikal voneinander beabstandete Lehnensegmente unterteilt sind, von denen zumindest einige, vorzugsweise jedoch alle, flexibel und/oder federnd aufgehängt und/oder an der Seitenwand oder Rückwand der Wärmekabine aufgehängt sind.

Eine Variante der Kabine kann vorsehen, dass das Multifunktionselement mit leitungsführenden Kanälen, Schächten etc. ausgebildet und insbesondere hohl, mit innenliegendem Hohlraum versehen ist. Der Hohlraum kann in Längsrichtung mindestens einmal unterteilt sein, zur Bildung von zwei oder mehr Schächten zur Aufnahme von Versorgungsleitungen aller Art wie bspw. Elektrokabel, Glasfaser, Luftschläuche etc., Aromaleitungen, Duftleitungen etc.

Das erfindungsgemäße Multifunktionselement kann gemäß einer vorteilhaften Variante mindestens eine Zuluftöffnung, mindestens eine Abluftöffnung und Leitungen für die Luftzufuhr und Luftabfuhr aufweisen. Die Leitungen für die Luftzufuhr und Luftabfuhr im Inneren des wenigstens einen Multifunktionselements sind geführt, d.h. mit definiertem Verlauf ausgestaltet. Das in die Wärmekabine eingepasste Multifunktionselement erfüllt mehrere technische Aufgaben wie Trage- und Haltefunktionen und stellt darüber hinaus eine Steuerungseinheit für die Wärmestrahler des Multifunktionselements bereit. Grundsätzlich kann dieses erfindungsgemäße Multifunktionselement die unterschiedlichsten Funktionen enthalten und Aufgaben erfüllen, wie nachfolgend noch näher zu erläutern sein wird. Neben den erwähnten Luftführungsaufgaben sind zahlreiche weitere Funktionen möglich und Teil der Erfindung.

Das Multifunktionselement kann insbesondere derart in der Wärmekabine eingebaut werden, dass es zwischen dem Boden und der Decke verläuft und dadurch ein statisches und ggf. stützendes Bauteil der Wärmekabine bildet. Das Multifunktionselement kann somit ein Last tragendes Bauteil bilden, das zur Deckenabstützung dienen kann. Eine mögliche Ausführungsform kann vorsehen, dass es den Boden und/oder die Decke durchdringt, so dass von außerhalb der Wärmekabine die Zu- und/oder Abfuhr der Luft und von elektrischer Energie durchführbar ist. Das eigentliche Multifunktionselement ist bei dieser Ausführungsvariante im Bereich innerhalb der Wärmekabine als geschlossenes und einheitliches Bauteil ausgebildet, während alle Anschlüsse von außen zugänglich bleiben.

Ebenso wäre es denkbar, dass das Multifunktionselement direkt in der Wärmekabine eingebaut wird, so dass es zwischen zwei im Wesentlichen gegenüber liegenden Punkten der Seitenwand verlaufen kann und dadurch wiederum ein statisches Bauteil der Wärmekabine bildet. Auch bei dieser möglichen Ausführungsform wäre es möglich, dass das Multifunktionselement beidseitig die Seitenwand durchdringt, so dass auch hier von außerhalb der Wärmekabine die Zu- und/oder Abfuhr der Luft und von elektrischer Energie durchführbar ist.

Grundsätzlich sind die unterschiedlichsten Einbaupositionen und Ausrichtungen des erfindungsgemäßen Multifunktionselementes in der Wärmekabine möglich, so senkrechte, waagrechte oder diagonale Verläufe. Wahlweise kann das Multifunktionselement dabei auch tragende Funktionen übernehmen. Das Multifunktionselement kann gerade oder gekrümmt ausgebildet sein.

Bei einer waagrechten oder schrägen Einbauvariante kann das Multifunktionselement als zusätzlicher Handlauf und/oder fest montierter oder schwenkbarer Handtuchhalter ausgebildet sein. Selbstverständlich ist es nicht unbedingt notwendig, dass das Multifunktionselement zwischen zwei gegenüber liegenden Wänden fixiert ist, sondern es kann auch einen U-förmigen Verlauf aufweisen und mittels Verankerungspunkten an der Decke und/oder am Boden der Wärmekabine befestigt sein. Bei einer solchen Variante kann zwischen vertikalen Abschnitten ein schräger oder horizontaler Abschnitt vorgesehen sein. Auch diagonale Verläufe des Multifunktionselements sind möglich, bei denen Seitenwände mit der Decke und/oder dem Boden verbunden sein können.

Dieses Multifunktionselement kann bspw. als ein Rundrohr ausgebildet sein, aber es wäre auch jegliche andere Form eines Rohres möglich. So kann es einen mehreckigen oder ovalen oder auch anderen, ggf. unregelmäßigen Querschnitt aufweisen. Ein derartiger unregelmäßiger Querschnitt kann bspw. eine oder mehrere Ausformungen zur Kabelführung o. dgl. vorsehen. Zudem sind auch Mehrkammernsysteme und/oder Querschnitte, die Versteifungsstege aufweisen, möglich. So kann der innen liegende Hohlraum des Multifunktionselements bzw. des Multifunktionsrohres in seiner Längsrichtung mindestens einmal durch eine Wandung unterteilt sein, so dass mindestens zwei parallel verlaufende Schächte gebildet sind, die ggf. unterschiedliche Funktionen erfüllen können.

Einer der wesentlichen Aspekt einer bevorzugten Ausführungsvariante der erfindungsgemäßen Wärmekabine ist durch die Funktion des Multifunktionselements als Luft führendes Element gegeben. So kann es einerseits der Zu- bzw. Abfuhr von Luft dienen, wozu eine im Inneren des Multifunktionselements ausgebildete Luftleitung oder längliche Luftkammer vorgesehen sein kann. Auch können die Zu- und/oder Abluftöffnungen entweder offen und/oder variabel verschließbar ausgebildet sein. Die Zu- und/oder Abluftöffnungen können grundsätzlich die unterschiedlichsten Ausgestaltungen aufweisen. So können sie bspw. als Gitter, als kreisrunde Öffnungen, als Öffnungsschlitze oder dgl. ausgebildet sein. Wahlweise können die Zu- und/oder Abluftöffnungen innerhalb und/oder außerhalb der Kabine, entweder seitlich am Rohr oder an einem der beiden Enden oder an beiden Enden des Rohres vorgesehen sein. Die Belüftungsfunktion kann wahlweise statisch sein, wobei ein Kamineffekt zur Luftführung der warmen Abluft von unteren Bereichen der Kabine nach oben, vorzugsweise zu Abluftöffnungen außerhalb der Kabine genutzt werden kann. Wahlweise oder zusätzlich kann eine mechanische Be- und/oder Entlüftung in Gestalt wenigstens eines Saug- oder Druckventilators vorhanden sein. Ein solcher Ventilator kann bspw. im Rohr oder auch außerhalb des Rohres bzw. des rohrförmigen Multifunktionselementes angeordnet sein.

Das Multifunktionselement kann gemäß einer weiteren Ausführungsvariante der Erfindung beweglich in der Wärmekabine verankert sein, so dass es insbesondere um seine Längsachse drehbar gelagert sein kann. Zu diesem Zweck kann es an seinen beiden Enden in Drehlagern befestigt sein, die in den Wänden, der Decke oder im Boden der Wärmekabine fixiert sind. Diese Drehlager können mit oder ohne Anschlagbegrenzung zur Begrenzung eines maximalen Drehwinkels versehen sein. Falls keine Anschlagbegrenzungen vorgesehen sind, kann es von Vorteil sein, Drehdurchführungen für elektrische Leitungen vorzusehen, so dass das Multifunktionselement in eine beliebige Winkelstellung gebracht werden kann, ohne dass dies durch die Verlegung elektrischer Leitungen innerhalb des Hohlraums des Multifunktionselements beeinträchtigt wird.

Das Multifunktionselement kann bspw. aus Metall, Kunststoff, Glas oder aus Verbundwerkstoff oder aus einer Kombination dieser genannten Materialien gefertigt sein. Als Verbundwerkstoffe kommen bspw. CfK oder GfK o. dgl. in Frage, die ggf. mit Einlagen oder Bestandteilen aus Metall oder Glas versehen sein können. Weiterhin kann es von Vorteil sein, wenn das Multifunktionselement mit einer hitzeresistenten und/oder isolierenden Beschichtung versehen ist. Dies kann bspw. eine Pulverbeschichtung, eine Kunststoffbeschichtung oder eine geeignete metallische oder metallhaltige Beschichtung sein. Eine weitere Variante kann vorsehen, dass das Multifunktionselement eine in Längserstreckungsrichtung um das Multifunktionselement gewickelte Metallwendel o. dgl. als Verbrennungsschutz für den Benutzer aufweist. Anstelle einer solchen wendelförmigen Schutzeinrichtung kann selbstverständlich auch ein anders gestalteter Berührschutz vorgesehen sein, bspw. ein gitterförmiger oder durch mehrere Längsstreben gebildeter Schutz zur Verhinderung einer direkten Berührung der Außenseite des Multifunktionselements.

Eine weitere vorteilhafte Ausführungsvariante sieht einen Duftspender oder Zerstäuber oder dgl. vor, der bspw. mittels eines Ventilators mit Frischluft von außen beaufschlagt werden kann, so dass an dem Duftspender vorbei geführte Frischluft mit einer definierbaren Menge eines Geruchs- oder Duftträgers beaufschlagt und anschließend in die Wärmekabine geleitet werden kann. Je nach Ansteuerung des Duftspenders bzw. Zerstäubers kann die Menge an in die Kabine geleitetem Geruchs- oder Duftträger reguliert werden. Dieser Zerstäuber kann im Zusammenhang mit der Luftabsaugung bzw. kaminartigen Luftabführung zur Verbesserung der Luftqualität innerhalb der Kabine beitragen, da bei deren häufiger Frequentierung und nicht ausreichender Belüftung die Atemluftqualität stark abnehmen und der CO₂-Gehalt ansteigen kann. Das erwähnte Beduftungssystem kann wahlweise integrierter Bestandteile des Multifunktionselements bzw. eines der Multifunktionselemente sein oder ein separates Element darstellen, das zweckmäßigerweise in eine Rück- oder Seitenwand oder in ein Deckenelement der Wärmekabine eingebaut ist und im Zusammenhang mit einer Frischluftzuführung zu sehen ist.

Neben seiner Eigenschaft als Luft führendes Element und als Tragstütze für die Infrarotstrahler kann das Multifunktionselement als Tragelement ausgebildet sein. Es ist für einen Fachmann selbstverständlich, dass die hier beschriebenen Ausführungsformen nicht als eine Beschränkung der Erfindung aufgefasst werden können. Verschiedene Formen des Multifunktionselements sind dabei denkbar, die eine statische und/oder funktionelle Aufgabe für die Wärmekabine erfüllen.

Für die individuelle und selektive Bestrahlung von diversen Körperteilen des Benutzers der Wärmekabine können ein oder mehrere Wärmestrahler nebeneinander im oder am Multifunktionselement angebracht werden. Auch können diese Wärmestrahler übereinander in verschiedenen Abständen im oder am Multifunktionselement montiert werden, um dadurch die Verwendung verschiedener Wärmestrahlertypen zu ermöglichen.

Die Bestrahlung von diversen Körperteilen wird intensiviert, wenn die Wärmestrahler horizontal nach allen Seiten schwenkbar und/oder vertikal neigbar ausgebildet werden können und zudem noch entlang der Länge des Multifunktionselements verschiebbar angebracht werden.

Um eine bessere Belüftung, insbesondere mit Umgebungsluft und eine Entlüftung der Wärmekabine zu ermöglichen, ist eine oder mehrere Zuluftöffnungen und Abluftöffnungen für das Innere der Wärmekabine an gegenüberliegenden Enden des Multifunktionselements angebracht.

Die Zu- und Abluft kann am Wärmestrahler im oder am Multifunktionselement vorbei geleitet werden. Eine solche Luftführung kann derart ausgestaltet sein, dass über Konvektion eine Kühlung der Wärmestrahler in oder am Multifunktionselement erzielbar ist. Die mechanische Luftführung kann bspw. mittels eines im Multifunktionselement montierten Impellers oder eines an zumindest einem Ende des Multifunktionselements montierten und schaltechnisch entkoppelten Ventilators erreicht werden. Durch das Vorsehen eines Druck- oder Saugfilters können die Wärmestrahler in oder am Multifunktionselement mit einer gerichteten Luftströmung gekühlt werden.

Die Ansteuerung von unterschiedlichen Betriebsparametern der Wärmestrahler des Multifunktionselements und damit der Temperatur- und Feuchtigkeitsbedingungen innerhalb der Wärmekabine kann über eine Bedieneinheit erfolgen. Diese Bedieneinheit kann fest oder lösbar mit dem Multifunktionselement verbunden sein. Die Steuerung erfolgt über die im Multifunktionselement integrierten Funktionen und/oder Parameter und diese sind somit einstellbar und/oder veränderbar. Die Bedieneinheit kann weitere Steuerungs- und/oder Anzeigefunktionen erfüllen, so z.B. die Lautstärkeregelung von Musikwiedergabe sowie von Bedienungsansagen und/oder Warnsignalen. Diese Wiedergaben können über mindestens einen im Multifunktionselement der Wärmekabine eingebauten Lautsprecher erfolgen. Durch die Integration des Lautsprechers in das Multifunktionselement ist dieser zudem noch gut gegen äußere Einflüsse geschützt.

Die Bedieneinheit ist zudem mit einer Steuerungseinheit verbunden. Die Steuerungseinheit dient zur Ansteuerung von mehreren Wärmestrahlern von Wärmekabinen, so dass bspw. die Wärmeleistung jedes einzelnen Wärmestrahlers vom Benutzer manuell einzeln reguliert werden kann. Dadurch, dass die Steuerungseinheit mit jedem einzelnen Wärmestrahler in der Wärmekabine kommunikativ verbunden ist, kann die Wärmeleistung mehrerer Wärmestrahler wahlweise auch gemeinsam oder eben einzelnen selektiv geregelt werden. Die Regelung der Wärmeleistung eines jeden einzelnen Wärmestrahlers ist eine manuelle und programmgesteuerte Regelung, d.h. dass die Wärmeleistung jedes einzelnen Wärmestrahlers vom Benutzer manuell reguliert werden kann.

Weitere Möglichkeiten können sich aus der Verbindung der Bedieneinheit mit der Steuerungseinheit ergeben, so bspw. die umfassende Zugriffsmöglichkeit auf Musik- und/oder Bildmedien, die auf geeigneten Speicherinhalten abgelegt sind. So kann die Bedieneinheit z.B. zur Musikwiedergabe ausgerüstet sein, wahlweise zum Datenempfang von der Steuerungseinheit und/oder zur Wiedergabe aus einem eigenen Speicher und/oder Datenträger. So kann bspw. in der Bedieneinheit ein MP3-Spieler o. dgl. integriert sein. Zudem können in der Bedieneinheit und/ oder in dem dieser zugeordneten Multifunktionselement Lautsprecher integriert sein, wobei das Multifunktionselement ggf. als Klangkörper genutzt werden kann.

Für die Betätigung der verschiedenen Funktionen mittels der Bedieneinheit sind bereits zahlreiche Prinzipien bekannt. So eignen sich bspw. berührungsempfindliche Bildschirme zur Eingabe von Steuerbefehlen. Daneben können Folientastaturen oder berührungsempfindliche Bedienfelder zur Benutzereingabe dienen. Derartige berührungsempfindliche Felder oder Bildschirme weisen den Vorteil auf, dass eine gegen Schmutz unempfindliche und leicht gegen Feuchtigkeitseinflüsse abzuschirmende Eingabetastatur zur Verfügung gestellt wird, die zudem je nach Bedarf grafisch gestaltet werden kann, um dem Benutzer die Betätigung zu erleichtern.

In der Wärmekabine kann ein Teil der Wärmestrahler als spezielle Rückenstrahler ausgebildet sein, die jeweils mit einem spektralen Filter versehen sind und eine gewölbte Form aufweisen; bspw. kann ein Robax® Filter verwendet werden, der durch das einseitige Aufbringen oxidischer, elektrisch leitfähiger Schichten den infraroten Strahlungsanteil und damit die Wärmestrahlung weitgehend reflektiert.

Ein weiterer Teil der Wärmestrahler können als Frontstrahler ausgebildet sein, die zur Richtung der Wärmestrahlung ebenfalls besonders geformt und mit einem spektralen Filter versehen sind. Der speziell geformte Reflektor dient zu Erzielung von unterschiedlichen Abstrahlwinkeln. Die Hauptanforderung des Frontstrahlers ist es, die Kernstrahlung an den Oberkörper und an die beiden Knie eines Benutzers zu reflektieren. Das Frontglas soll den schädlichen Teil des Spektrums filtern, um ohne Gefahr für die Augen in den Frontstrahler blicken zu können.

Die erfindungsgemäße Wärmekabine kann in ihrer einfachsten Bauform wenigstens einen Infrarotstrahler aufweisen. Die Bestrahlungswirkung lässt sich jedoch besser an die Wünsche und Bedürfnisse eines Benutzers anpassen, wenn mehrere Infrarotstrahler vorhanden sind bzw. wenn eine besondere Gehäuse- und Reflektorbauweise für Front- und/oder Rückenstrahler aus einer besonderen Kombination der Reflektorform, der Reflektorbeschichtungen, der Strahlerpositionierungen und ggf. der Lichtaustrittsflächen der Strahler zum Einsatz kommt. So kann das bereits erwähnte sog. Robax®-Glas mit teilweiser oder kompletter Beschichtung, deckend oder transluzent oder unbeschichtet und/oder mit anderen spektralen Filtereigenschaften zum Einsatz kommen, um auf diese Weise ein optimiertes Strahlungsspektrum (Infrarot-A-, -B- und/oder -C-Strahlung) zu erhalten.

Die gesamte Strahlerausstattung mit ggf. mehreren Infrarotstrahlereinheiten für Front- und/oder Rückenstrahler richtet sich nach den Erfordernissen und kann nahezu beliebige Kombinationen von verschiedenen Reflektorformen, von Reflektoranordnungen und/oder Aufhängungspunkten der Reflektoren, von Lichtaustrittsflächen - z.B. ganz oder teilweise beschichtet, deckend oder transluzent beschichtet oder unbeschichtet -für einen gewünschten Bestrahlungsbereich vorsehen. So können die genannten Ausstattungen je nach Austrittswinkel bzw. Abdeckungsbereich der Strahlung differieren. Auch können die Ausstattungen je nach gewünschtem Strahlungsspektrum differieren.

Es können grundsätzlich zahlreiche Varianten von starr platzierten und/oder höhenverstellbaren und/oder winkel- und neigungsverstellbaren Reflektoren eingesetzt werden. Dies betrifft alle vorhandenen Reflektoren, seien dies Frontstrahler, Seitenstrahler oder Rückenstrahler. Ggf. können einzelne oder alle Reflektoren stufenlos in alle Richtungen verschwenkbar ausgebildet sein. Wahlweise können auch zwei oder mehr Infrarotstrahler übereinander angeordnet sein.

In der Wärmekabine können weiterhin Reflektorfolien vorhanden sein, insbesondere an der Seitenwand, am Boden und/oder der Decke. Hierbei handelt es sich vorzugsweise um Reflektorfolien, welche seitlich an der Kabinenwand, am Boden und/oder der Kabinendecke vorgesehen sind, um die Infrarotstrahlung der Front- und Rückenstrahler auf den menschlichen Körper zu lenken und dadurch eine gleichmäßige Erwärmung zu erzielen. Diese Anwendung dient vor allem für den seitlichen Gesäßbereich in Sitzposition, aber auch für den Wadenbereich.

Die Wärmestrahler in der Wärmekabine können gemäß einer bevorzugten Ausführungsform der Erfindung bspw. als Keramikstrahler ausgebildet sein. Des Weiteren besitzt der Keramikstrahler eine Füllung, die aus einer Mischung aus Lavasand und Quarzsand besteht, wobei die Füllung zum Wärmetransport und Wärmespeicherung von einer Heizwendel zur Oberfläche des Keramikstrahlers dienen soll. Mit dieser Zusammensetzung wird die Erwärmung im System beschleunigt. Wahlweise können auch Infrarotlampen mit besonderen Mischungen aus Quarz- und/oder Lavasand zur Erzielung einer bereits unmittelbar nach dem Einschalten wirksamen und lang anhaltenden Infrarotstrahlung zum Einsatz kommen.

Ein Einstieg in die Wärmekabine erfolgt zweckmäßigerweise frontal oder seitlich zu einer in der Wärmekabine angeordneter Sitzbank. Vor der Sitzbank kann eine Seitenwand als Glasfront zur Aussicht dienen. Bei dieser Glasfront kann es sich um ein transparentes, wahlweise jedoch auch um transluzentes Material handeln. In Abgrenzung zur Transparenz kann man Transluzenz als Lichtdurchlässigkeit beschreiben und Transparenz als Bild- oder Blickdurchlässigkeit. Selbstverständlich können unterschiedliche Varianten und Grundrisse der Kabine realisiert sein, die allesamt von der vorliegenden Erfindung mit umfasst sind. So kann die Kabine eine gerade, eine halbrunde oder leicht gewölbte Frontseite mit zentraler Tür aufweisen. Die Frontseite kann wahlweise auch geschlossen ausgebildet sein, so dass sich die als Einstieg ausgebildete Tür seitlich, d.h. an einer Schmalseite der Wärmekabine befindet.

Von besonderem Vorteil kann es weiterhin sein, wenn der Kabinenboden derart seitlich nach oben gezogen ist, dass eine flüssigkeitsdichte Wanne gebildet ist, die sich besonders für einen Dauereinsatz und/oder für eine Dauerbelastung im gewerblichen Einsatz eignet. Auf diese Weise kann die Wärmekabine auch bei hoher Benutzungsfrequenz und der damit einhergehenden Problematik der Entstehung von relativ viel Feuchtigkeit und Nässe leicht gereinigt und ohne das Risiko einer Beschädigung aufgrund einer Durchfeuchtung von Holzelementen o. dgl. benutzt werden. Dieser geschlossene Wannenboden bietet besonders bei Wärmekabinen aus Holz besondere Vorteile, da diese nicht mit einem zu hohen Feuchtigkeitsanfall konfrontiert werden können, ohne besondere Konservierungs- und/oder Versiegelungsmaßnahmen zu treffen. Auch können mit einer solchen geschlossenen Wanne geltende Hygienevorschriften wesentlich leichter eingehalten werden, da anhaftende Mikroorganismen problemlos abgewaschen werden können und sich nicht in der organischen Holzoberfläche festsetzen können.

Durch die erfindungsgemäße Gestaltung kann ein sauberer Umgang mit der Kabine gewährleistet werden, da Flüssigkeiten wie Schweiß, Wasser etc. gesammelt und ggf. nach außen geführt werden können. Die Wanne kann aus unterschiedlichen Materialen gebildet sein, bspw. aus Kunststoff, aus Metall, ggf. mit zusätzlicher Beschichtung o. dgl., während die Wände und/oder Decke der Kabine aus Holz und/oder Glas gebildet sein können. Selbstverständlich kann die Wannenform auch auf andere Weise realisiert sein, bspw. durch entsprechende feuchtigkeitsresistente Wandabschnitte im unteren Bereich der Kabine, die mit einem feuchtigkeitsresistenten Bodenbereich in geeigneter Weise, d.h. dicht verbunden sein können. Wichtig dabei ist, dass die Kabinenwände in keinem direkten Feuchtigkeitskontakt stehen.

Es kann weiterhin auch mindestens eine Liege bzw. ein Liegesitz in der Wärmekabine vorhanden sein. Die Liege wird vorzugsweise hohl oder als Schale ausgebildet und kann aus sämtlichen Materialien hergestellt werden. Die Ausgestaltung der Liege kann beweglich oder starr sein. Eine individuelle Ausgestaltung der Sitzposition wird dadurch ermöglicht, indem das Körpergewicht auf eine möglichst große Aufliegefläche verteilt wird, somit werden keine Druckstellen verspürt und es wird ein besserer Sitz-Liegekomfort gewährleistet. Zudem können die Liegen bzw. die Liegesitze auch kippbar, ergonomisch und/ oder beheizbar ausgestaltet werden.

Eine weitere Ausführungsform wäre ein ergonomischer Lehnenteil, welcher sich dreidimensional bewegt und somit sich den Rücken besser anpasst und die Sitzqualität wesentlich verbessert. Dieser Lehnenteil kann sowohl bei Flächenstrahlern als auch bei Röhrenstrahlern in Einsatz kommen. Die Position des Strahlers nicht ausschlaggebend. Er kann in die Rückwand gerade oder schräg eingebaut werden.

Eine weitere bevorzugte Ausführungsvariante kann eine gefederte und/oder bewegliche Rückenlehne und/oder Sitzschale vorsehen, die ggf. zwei getrennte Abschnitte aufweisen kann, so dass eine optimale und ungehinderte Bestrahlung des gesamten Rückens eines Benutzers im Wirbelsäulenbereich ermöglicht wird. Diese mehrteilige Rückenlehne kann bspw. zwei annähernd vertikal verlaufende Abschnitte aufweisen, zwischen denen ein Strahler angeordnet sein kann. Diese annähernd vertikal verlaufenden Abschnitte können wahlweise jeweils einteilig ausgebildet und vorzugsweise einen ergonomisch günstigen gewölbten Verlauf aufweisen. Die annähernd vertikal verlaufenden Lehnenabschnitte können jedoch in einer vorteilhaften Ausführungsvariante segmentiert ausgebildet sein und aus einer Mehrzahl von gleichartigen oder verschieden großen, jeweils rechteckförmigen und vorzugsweise separat federnd und gelenkig gelagerten Lehnensegmenten gebildet sein. Wahlweise können diese Segmente flache oder leicht nach außen gewölbte Oberflächen aufweisen. Weiterhin kann es von Vorteil sein, die gesamten Lehnenabschnitte und/oder den gesamten Sitzbereich verschwenkbar auszubilden, um zwischen einer aufrechten und einer liegenden Ruheposition verschiedene Einstellungen realisieren zu können.

Die erfindungsgemäßen Sitz- und/oder Lehnenelemente können wahlweise aus Holz- und/oder Kunststoffmaterial gebildet sein, vorzugsweise jedoch aus Materialien, die eine geringe Wärmeleitfähigkeit aufweisen, um dem Benutzer auch bei einer Auflage mit bloßer Haut jederzeit ein angenehmes Sitzgefühl bieten zu können, ohne dass es zu lokalen Temperaturspitzen kommt, die bei längerer Benutzung unangenehm wären. Als Material für die Liegen und/oder Lehnenteile kommen neben Holz und Kunststoff auch Glas, Keramik, Polyester oder ähnliche Materialien synthetischer oder natürlicher Herkunft in Betracht.

Wahlweise können mit dem System die Sitze bzw. Sitzliegen für eine Ein-, Zwei- oder Mehrsitzerkabine ausgestaltet sein. So können die Sitze bzw. Sitzliegen aus einer durchgehenden oder mehrteiligen Sitzbank o. dgl. gebildet sein.

Wahlweise können auch besonders ergonomisch gestaltete Sitz- und/oder Lehnenelemente in der Wärmekabine vorgesehen sein, bspw. ergonomische Sitze mit mehreren Segmenten und/oder mit Netzlehnen, bei denen sich die Bezüge leicht entnehmen und reinigen lassen.

Eine weitere besondere Anwendung sind Einrichtungen zur Erzeugung von Magnetfeldern, die zum Teil in den Liegen bzw. Sitzliegen vorhanden sein können. Wahlweise können solche Einrichtungen auch in der Wärmekabine selbst vorgesehen sein. Derartige Einrichtungen können zur gezielten Anwendungsvariation für unterschiedliche Bedürfnisse der Benutzer der Wärmekabinen dienen, insbesondere in Kombination mit den Wärme- bzw. Infrarotstrahlern.

Ein bisher nicht erwähnter, jedoch ebenfalls im Zusammenhang mit der vorliegenden Erfindung zu sehender Aspekt betrifft die Beleuchtung der Wärmekabine. Hierzu eignen sich Lichtsysteme zum Wand- und/oder Deckeneinbau und/oder zur Integration in die Multifunktionselemente bzw. -säulen. Solche Lichtsysteme können die unterschiedlichsten Leuchtmittel aufweisen, bspw. Licht emittierende Dioden, Halogenstrahler und/oder Gasentladungslampen o. dgl. Das Abstrahlverhalten und die abgestrahlten Wellenlängenbereiche können in gewünschter Weise definiert werden, um den Benutzern eine angenehme und die Entspannung fördernde Beleuchtung zu bieten. Auch können die Wellenlängenbereiche und damit die Lichtfarben ggf. vom Benutzer beeinflusst werden, ebenso wie die Lichtintensitäten. Es sind nahezu beliebige Kombinationen der erwähnten Lichtelemente bzw. Leuchtmittel in allen erwähnten Einbaupositionen möglich, um die gewünschte Beleuchtung und den gewünschten optischen Eindruck zu erzielen.

Ein ebenfalls bisher nicht erwähnter, jedoch im Zusammenhang mit der vorliegenden Erfindung zu sehender Aspekt betrifft die äußere Gestaltung der Wärmekabine, die in einer vorteilhaften Ausführungsvariante mit den unterschiedlichsten Flächenelementen ausgestattet sein kann. Hierfür eignen sich insbesondere flächige Dekorelemente aus Holz, Kunststoff, mineralischem Material und/oder aus Verbundmaterialien, die in einer vorteilhaften Variante ggf. austauschbar und damit leicht von den Außenwänden entnehmbar und wieder fixierbar ausgestaltet sein können. Diese Dekorelemente können somit in einem Baukastenprinzip in den unterschiedlichsten Varianten zur Verfügung gestellt werden, so dass je nach Designwunsch des Benutzers oder Betreibers der Wärmekabine die unterschiedlichsten äußeren Erscheinungsformen ermöglicht sein können.

Es sei an dieser Stelle zusätzlich darauf hingewiesen, dass die Wärmekabine nicht nur ein einziges Multifunktionselement aufweisen kann, sondern wahlweise auch zwei, drei oder mehr gleichartige oder unterschiedliche Multifunktionselemente. Diese können wahlweise parallel angeordnet sein und z.B. senkrecht zwischen Decke und Boden verlaufen. Es können jedoch auch mehrere Multifunktionselemente in jeweils unterschiedlicher räumlicher Ausrichtung vorhanden sein.

Darüber hinaus ist klarstellend darauf hinzuweisen, dass der in der vorliegenden Anmeldung weitgehend durchgängig gewählte und verwendete Begriff "Multifunktionselement" in erster Linie verdeutlichen soll, dass es sich bei dem Element um ein multifunktionales, nicht auf eine bestimmte Funktion festgelegtes Bauteil handelt. Es ist damit jedoch nicht ausgedrückt, dass zwingend mehrere Funktionen - bspw. die Luftführung, die Befestigung der Wärmestrahler, die Befestigung des Bedien- und Anzeigepults etc. - darin enthalten oder damit verbunden sind. Prinzipiell könnten die tragenden oder nichttragenden Säulen und/oder Querverstrebungen mit den darin verlaufenden Luftkanälen auch anders bezeichnet werden, bspw. als Luftleitungskanäle, als Stützelemente, als Strahlerelemente oder dergleichen. Sollte sich zu einem späteren Punkt herausstellen, dass der Begriff "Multifunktionselement" besser durch einen der genannten Alternativbegriffe ersetzt werden sollte, so sind die oben gemachten Hinweise ausdrücklich so zu verstehen, dass der in der Anmeldung verwendete Begriff "Multifunktionselement" durchgängig durch einen der Alternativbegriffe erstetzt werden kann.

Nachfolgend sind nochmals die unterschiedlichsten Aspekte, Varianten und Ausführungsbeispiele der erfindungsgemäßen Infrarotkabine bzw. Wärmekabine mit dem wenigstens einen darin angeordneten Multifunktionselement aufgelistet bzw. dargestellt. Es ist mindestens ein Multifunktionselement vorhanden, das bspw. derart in die Wärmekabine eingebaut ist, dass es wie eine Säule und/oder Steher zwischen dem Boden und der Decke verläuft und dadurch ein statisches Bauteil zur Abstützung der Decke der Wärmekabine bildet. Eine Variante ist gekennzeichnet durch mindestens ein Multifunktionselement, das derart in die Wärmekabine eingebaut ist, dass es diagonal zwischen dem Boden und der Kabinenwand und/oder diagonal zwischen der Kabinenwand und der Decke und/oder waagrecht zwischen zwei gegenüberliegenden oder aneinander grenzenden Wandabschnitten der Kabinenwand verläuft und dadurch ein statisches Bauteil der Wärmekabine bildet. Das Multifunktionselement kann einen geraden oder gebogenen Verlauf aufweisen. Es kann einen kreisrunden, ovalen, eckigen oder sonstigen Querschnitt mit unterschiedlichen Maßen aufweisen, wobei vorzugsweise Rundrohr und/oder Formerohr in Frage kommt. Das Multifunktionselement kann über seine gesamte Länge oder Teile davon hohl ausgeführt sein. Das hohle Multifunktionselement kann einen innenliegenden Hohlraum bilden, der in der Längsrichtung mindestens einmal unterteilt ist und somit mindestens zwei parallel verlaufende Schächte aufweist. Bei dem hohlen Multifunktionselement kann mindestens eine Öffnung an mindestens einem Ende des Multifunktionselements vorhanden sein. Das Multifunktionselement kann als Handlauf und/oder Handtuchhalter ausgebildet sein.

Eine weitere Variante ist gekennzeichnet durch mindestens ein um die Längsachse drehbar gelagertes Multifunktionselement, dessen Drehlager mit oder ohne Anschlagsbegrenzung ausgeführt sind. Das Multifunktionselement kann um die Längsachse drehbar gelagert sein. Das Multifunktionselement kann aus Metall, Kunststoff, Glas und/oder Verbundwerkstoffen wie karbonfaserverstärkte Expoxid-Kunststoffe bestehen bzw. gefertigt sein. Weiterhin kann das Multifunktionselement mit einer hitzeresistenten und/oder isolierenden Beschichtung (insbesondere Pulverbeschichtung, Kunststoffbeschichtung oder Metallbeschichtung) versehen sein und/oder einer in der Längsrichtung um das Multifunktionselement gewickelten Metallspirale als Verbrennungsschutz für den Benutzer aufweisen.

Weiterhin ist vorzugsweise mindestens ein Infrarotstrahler (insbesondere Röhrenstrahler) mit oder ohne spektralem Filter für Infrarotstrahlung vorgesehen, der im/am Multifunktionselement angeordnet ist; dieses kann wahlweise auch mindestens zwei nebeneinander und/oder übereinander positionierten Strahlergehäuse für Infrarotstrahler aufweisen. Das Strahlergehäuse kann horizontal schwenkbar ausgebildet sein. Es kann auch ein vertikal neigbares Strahlergehäuse für Infrarotstrahler vorgesehen sein. Es können auch um eine und/oder mehrere Achse(n) drehbare Strahlergehäuse für Infrarotstrahler vorgesehen sein. Das Strahlergehäuse kann in Längsrichtung (höhenverstellbar) sein. Das Multifunktionselement kann ein stufenlos und/oder gerastert verstellbares Strahlergehäuse für Infrarotstrahler vorsehen. Eine weitere Variante ist gekennzeichnet durch mindestens ein Multifunktionselement mit Strahlergehäuse, das mindestens einen Griff aus hitzeresistenten und isolierenden Material zum Drehen des Multifunktionselementes und/oder des Strahlergehäuses aufweist.

Eine weitere vorteilhafte Variante ist gekennzeichnet durch ein hohles Multifunktionselement, das mindestens eine Öffnung mit beliebigen Öffnungsformen und Öffnungsgrößen am Ende des Multifunktionselements und/oder an dessen Seitenwand für die Einleitung und/oder Ausleitung von Versorgungsleitungen aller Art (wie Elektrokabeln, Glasfaserkabeln oder Luftschläuchen) sowie mindestens einen Schacht für deren Führung aufweist. Zudem kann ein hohles Multifunktionselement mit einem oder mehreren Luftschächten für die Belüftung und/oder Entlüftung der Wärmekabine versehen sein, wobei das Multifunktionselement an mindestens einer Stelle die Kabine (vorzugsweise das die Decke) nach Außen durchdringt und mindestens zwei getrennte Öffnungen mit beliebigen Öffnungsformen und Öffnungsgrößen am Ende des Multifunktionselements und/oder an dessen Seitenwand aufweist. Zudem kann ein hohles Multifunktionselement für die Belüftung und/oder Entlüftung der Wärmekabine ausgestattet sein, wobei die Lüftung statisch und/oder mechanisch (mittels eines elektrischen Ventilators) erfolgen kann. Das hohle Multifunktionselement kann für die Belüftung und/oder Entlüftung der Wärmekabine mit mindestens zwei manuell und/oder programmgesteuert verschließbaren Luftöffnungen versehen sein. Das hohle Multifunktionselement für die Belüftung und/oder Entlüftung der Wärmekabine kann wahlweise mit mindestens zwei manuell und/oder programmgesteuert verschließbaren Öffnungen mit gleichen und/oder unterschiedlichen Öffnungsformen und/oder Öffnungsgrößen für Zuluft und/oder Abluft (vorzugsweise am an beiden Ende des Multifunktionselements) versehen sein. Das hohle Multifunktionselement für die Belüftung und/oder Entlüftung der Wärmekabine kann wahlweise mit mindestens einem Infrarotstrahler ausgerüstet sein, wobei die Luftführung im Multifunktionselement derart ausgestaltet ist, dass über die thermische Konvektion der Luft (Kamineffekt) mit oder ohne mechanische Unterstützung (mittels eines elektrischen Ventilators) eine dynamische Kühlung des Infrarotstrahlers erreicht wird. Auch kann das hohle Multifunktionselement für die Belüftung und/oder Entlüftung der Wärmekabine so ausgestaltet sein, dass die Lüftung mit oder ohne mechanischen und/oder elektrischen Luftfilter zur Reinigung der Kabinenluft erfolgt. Weiterhin kann ein eingebauter manuell und/oder programmgesteuert bedienbarer Duftzerstäuber insbesondere für ätherische Essenzen vorhanden sein.

Eine weitere vorteilhafte Variante ist gekennzeichnet durch ein hohles Multifunktionselement mit eingebautem Lautsprecher samt Verkabelung zur Wiedergabe von Musik, Tönen, Bedienungsansagen und/oder Warnsignalen. Eine Variante ist gekennzeichnet durch ein Multifunktionselement mit einem eingebauten und/oder seitlich fest oder abnehmbar befestigten Bedienpaneel (Bedieneinheit) für die manuelle Steuerung der einzelnen Kabinenfunktionen und/oder Parameter, wobei das Bedienpaneel auch als Touchscreen ausgeführt sein kann. Das Multifunktionselement kann mit einem eingebauten und/oder seitlich fest oder abnehmbar befestigten LCD-Screen für die visuelle Anzeige von Bilddaten betreffend Kabinenparameter (wie intuitive Benutzerführung, Temperatur, Strahlerleistung und/oder Warnhinweisen) und/oder Videofilmen versehen sein.

Zudem kann eine Variante des Multifunktionselements eine eingebaute und/oder seitlich befestigte Lichtquelle samt Gehäuse und Verkabelung aufweisen, wobei die Lichtquelle als monochrome und/oder färbige LED, Halogenlampe, UV-Strahler oder sonstige Lichtquelle ausgeführt ist und manuell und/oder programmgesteuert geregelt wird. Weitere Details können sein:
- ein Aroma- & Frischluftsystem;
- ein Frischluftsystem (Lüftung, Kühlung & Aromatisierung) (besondere Luftzirkulation in der Kabine Rückwand und Multifunktionselement) Aroma- & Frischluftsystem (ermöglicht Einsaugen der Bodennahen Luft und Ausströmen der Luft über Kabinendecke);
- eine zentrale, elektronische Steuereinheit etc.

Die Steuereinheit dient innerhalb der Kabine zur Steuerung aller Funktionen, so dass die Wärmestrahler, Musik, Aroma und LED-Beleuchtung zentral gesteuert werden können (inkl. selektiver Steuerung der einzelnen Strahler) mit Sicherheitseinrichtung (automatische Zeitabschaltung für Kabine und Wärmestrahler sowie temperaturabhängige Abschaltung für Wärmestrahler). Vorteilhaft kann darüber hinaus sein:
- eine Einzelstrahlersteuerung;
- eine sensorgesteuerte Messung der Haut- und Kabinentemperatur für die Strahleransteuerung;
- ergonomische Sitze; bpsw. ergonomische Sitze mit flexibler Sektionallehne, klappbaren, ergonomischen Armlehnen und höhenverstellbaren Kopflehnen;
- Netzsitz;
- Seiteinstieg, insbesondere für besondere Raumverhältnisse;
- Flüssigkeitsdichter Wannenboden;
- flüssigkeitsdichter Kabinenboden in Wannenform;
- Reflektierende Seitenwände;
- Beschichtete Gläser und Wände mit Reflektorfolien zur Reflektion der IR-Wärmestrahlung innerhalb der Kabine (rasches Aufheizen und keine übermäßige Abkühlung bei geringer Betriebsleistung);
- Austauschbare Dekorfronten;
- Austauschbare Dekor-Fronten im Baukastenprinzip; etc.

Weitere Merkmale, Ziele und Vorteile der vorliegenden Erfindung gehen aus der nun folgenden detaillierten Beschreibung einer bevorzugten Ausführungsform der Erfindung hervor, die als nicht einschränkendes Beispiel dient und auf die beigefügte Zeichnung Bezug nimmt.

Fig. 1a-g zeigen Perspektivansichten unterschiedlicher Ausführungsformen einer beanspruchten Wärme- bzw. Infrarotkabine.

Fig. 2 zeigt eine Perspektivansicht einer weiteren Ausführungsform einer beanspruchten Wärmekabine.

Fig. 3a - b zeigen schematische Ansichten eines Multifunktionselements, das integraler Bestandteil einer Wärmekabine sein kann.

Fig. 4 zeigt schematische Ansichten von Möglichkeiten zur Positionsveränderung von Wärmestrahlern einer beanspruchten Wärmekabine.

Fig. 5 zeigt eine Draufsicht einer möglichen Anordnung von Wärmestrahlern in einer Wärmekabine.

Fig. 6 zeigt eine Draufsicht eine weitere mögliche Anordnung von zwei Wärmestrahlern in einer Wärmekabine.

Fig. 7a bis 7c zeigen Querschnitte von Wärmestrahlern in unterschiedlichen Positionen.

Fig. 7d - e zeigen Querschnitte von Wärmestrahlern und dem Multifunktionselement in unterschiedlichen Positionen.

Fig. 8 zeigt einen Querschnitt durch einen Wärmestrahler, wie er in einer beanspruchten Wärmekabine vorhanden sein kann.

Fig. 9a bis 9d zeigen mögliche Ausführungsformen einer Infrarotlampe, wie diese als Bestandteil eines Wärmestrahlers ausgestaltet werden können.

Fig. 10a zeigt eine schematische Frontalansicht und Fig. 10b zeigt eine schematische Seitenansicht einer möglichen Anordnung eines Rückenelements.

Fig. 11 zeigt eine schematische Seitenansicht einer möglichen Ausführungsform eines Lehnelementes, das integraler Bestandteil einer beanspruchten Wärmekabine sein kann.

Fig. 12 zeigt eine schematische Seitenansicht einer möglichen Ausführungsform eines Lehnelementes, das integraler Bestandteil einer beanspruchten Wärmekabine sein kann.

Fig. 13a bis 13c zeigen schematische Ansichten eines Duftzerstäubers, wie er in einer beanspruchten Wärmekabine vorhanden sein kann.

Fig. 14 zeigt mögliche Ausführungsformen zur Ausgestaltung von Bedieneinheiten, durch die der Betrieb einer beanspruchten Wärmekabine geregelt werden kann.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar. Vielmehr ist eine Vielzahl von Varianten und Abwandlungen denkbar, die von dem erfindungsgemäßen Gedanken Gebrauch machen und deshalb ebenfalls in den Schutzbereich fallen.

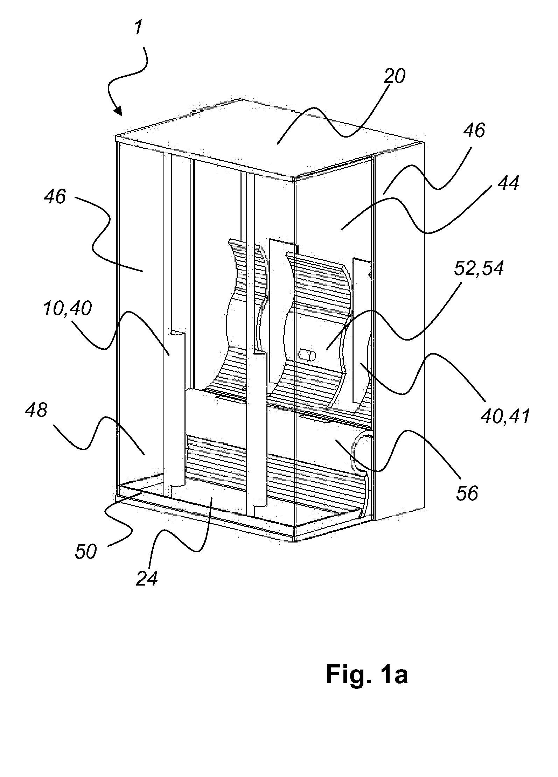

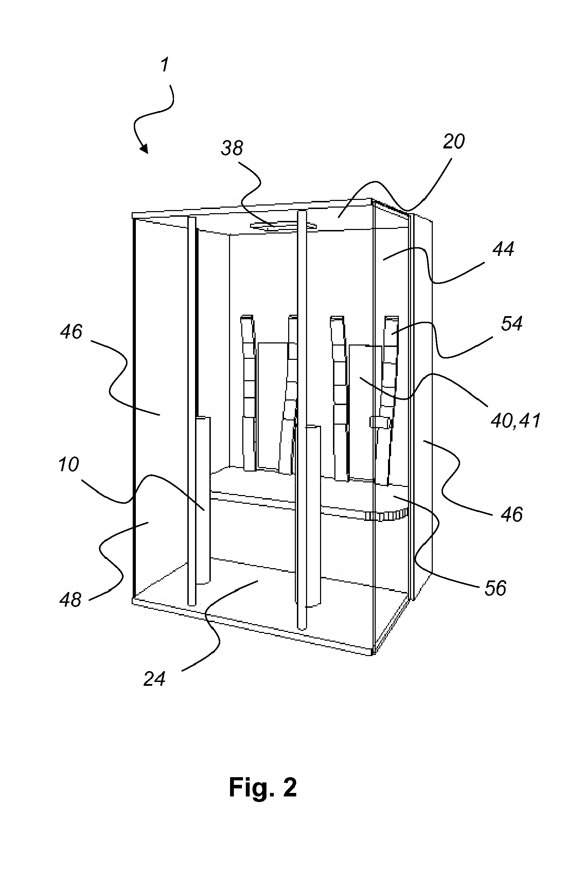

Die Fig. 1a-g und 2 zeigen Perspektivansichten von unterschiedlichen Ausführungsformen einer beanspruchten Wärmekabine 1, die im Folgenden auch als Infrarotkabine bezeichnet werden kann. Die Wärmekabine 1 wird von einer Decke 20, von einem Boden 24 sowie von Seitenwänden 46 und einer Rückwand 44 und einer Vorderwand 48 gebildet. Die umlaufenden Seitenwände 46 oder auch in der Vorderwand 48 kann eine Tür (nicht dargestellt) eingefasst sein, um in die Wärmekabine 1 einsteigen zu können.

An der Rückwand 44 können ergonomische Liegeelemente 52 angebracht sein. Diese Liegeelemente 52 können eine ganze Einheit bilden oder als Rückenelement 54 und Sitzelement 56 einzeln ausgebildet werden. Zwischen den Rückenelementen 54 sind Wärmestrahler 40 angebracht, die als Infrarotstrahler 41 ausgebildet sein können und speziell für die Erwärmung des Rückens dienen. Derartige Wärmestrahler 40 können auch in mindestens einem Multifunktionselement 10 integriert sein und/ oder als Infrarotstrahler 41 für den vorderen Bereich des Körpers dienen sowie an den Seitenwänden 46 der Wärmekabine 1 angebracht sein, damit diese Wärmestrahler 40 als Infrarotseitenstrahler (nicht dargestellt) dienen. Im vorliegenden Ausführungsbeispiel wird das Multifunktionselement 10 senkrecht zwischen der Decke 20 und dem Boden 24 der Wärmekabine 1 eingespannt.

An der Decke 20 können unterschiedlich ausgestaltete Beleuchtungseinrichtungen 38 (vgl. Fig. 2) angebracht sein. Auch besteht die Möglichkeit, dass diese Beleuchtungseinrichtungen 38 an den Seitenwänden 46 oder im Bodenbereich 24 der Wärmekabine 1 eingebaut sind (nicht dargestellt).

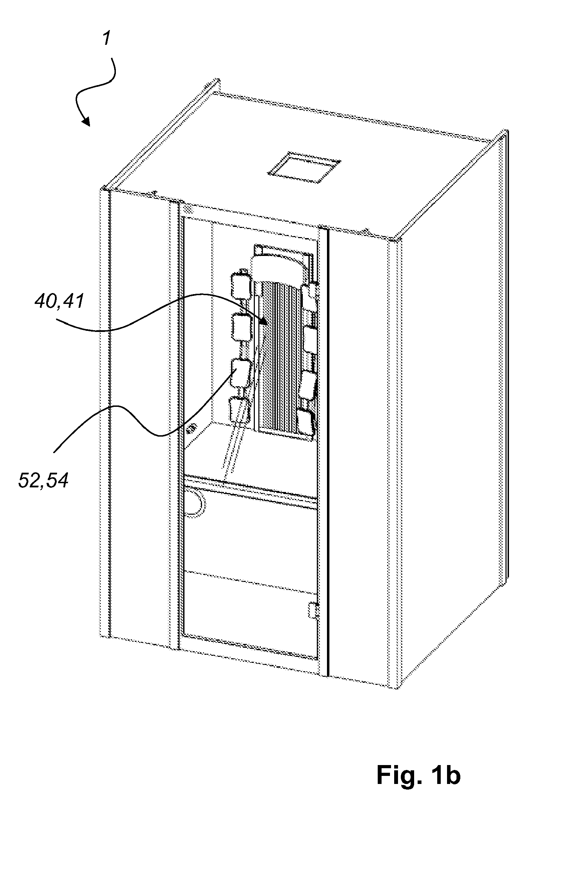

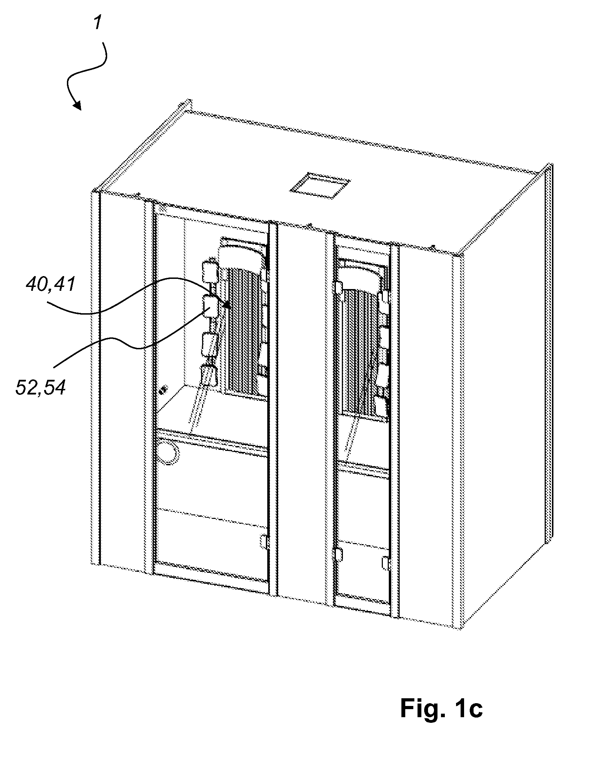

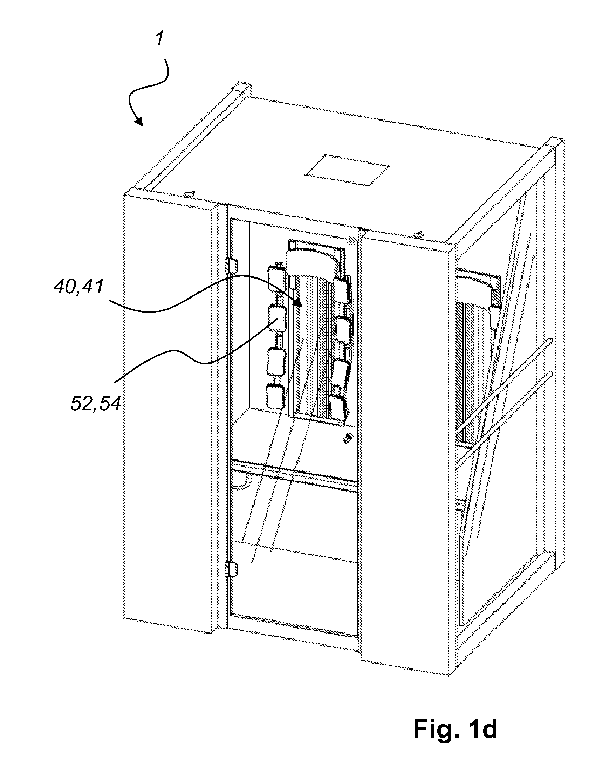

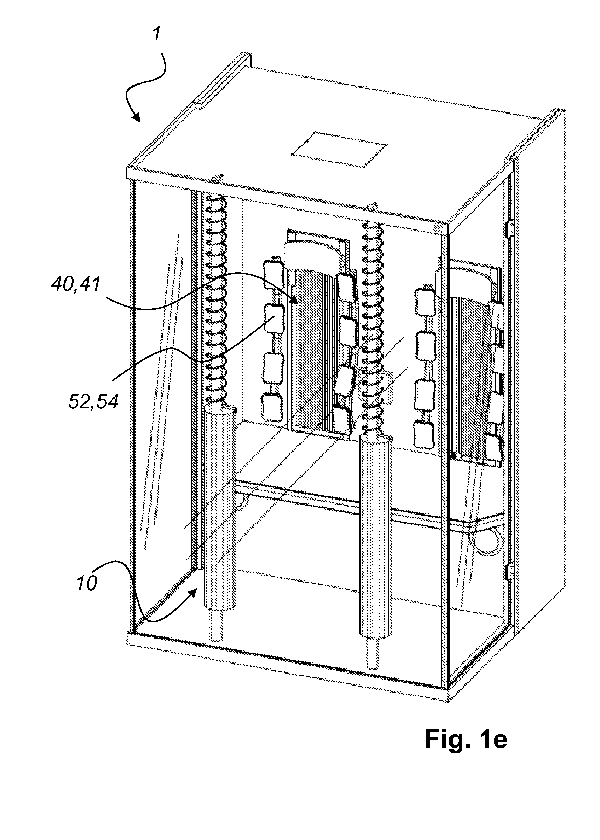

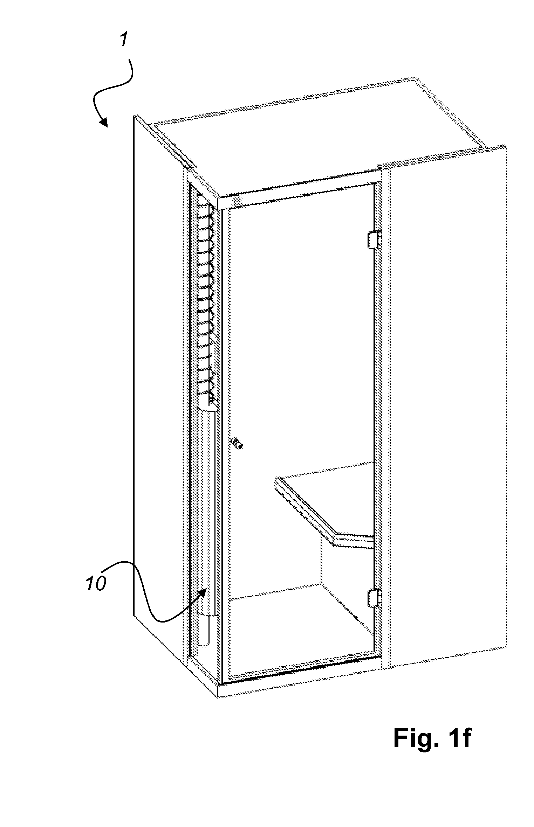

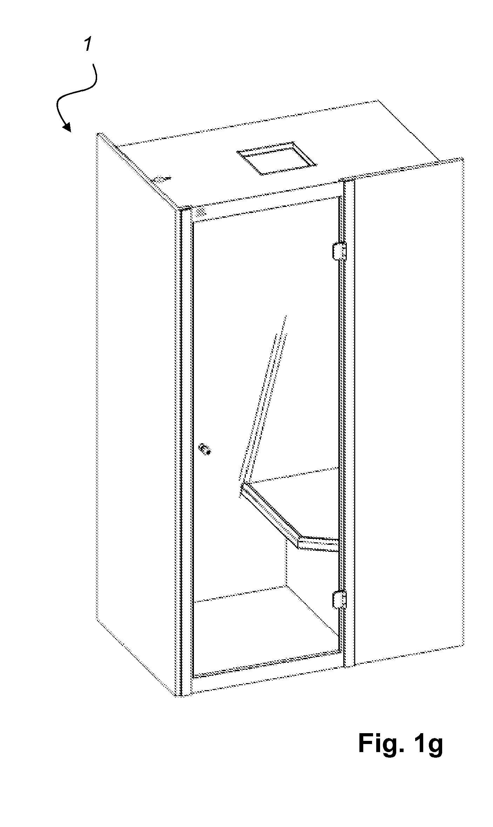

Der Boden 24 der Wärmekabine 1 umfasst zudem eine Wanne 50. In dieser Wanne 50 wird die Feuchtigkeit gesammelt, die während der Benutzung der Wärmekabine 1 sich bildet. Einen besonderen Vorteil kann es sein, wenn der Kabinenboden 24 derart seitlich nach oben gezogen ist (vgl. Fig. 1), dass die flüssigkeitsdichte Wanne 50 gebildet wird.

Durch die erfindungsgemäße Gestaltung kann ein sauberer Umgang mit der Kabine 1 gewährleistet werden, da Flüssigkeiten wie Schweiß, Wasser etc. gesammelt und ggf. nach außen über Abflussleitungen (nicht dargestellt) geführt werden können. Die Wanne 50 kann aus unterschiedlichen Materialen gebildet sein, bspw. aus Kunststoff, aus Metall, ggf. mit zusätzlicher Beschichtung oder dergleichen, während die Wände 46 und/oder Decke 20 der Kabine 1 aus Holz und/oder Glas gebildet sein können.

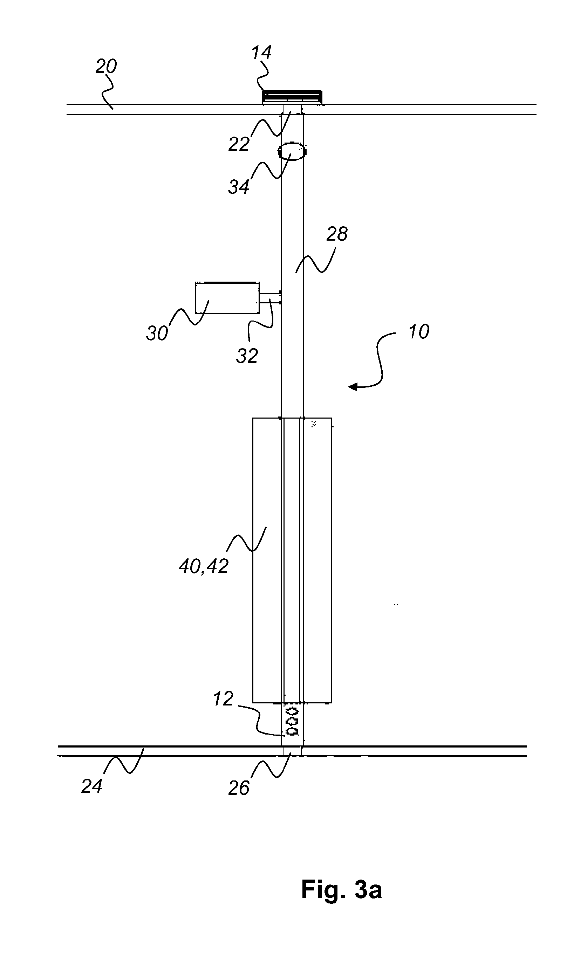

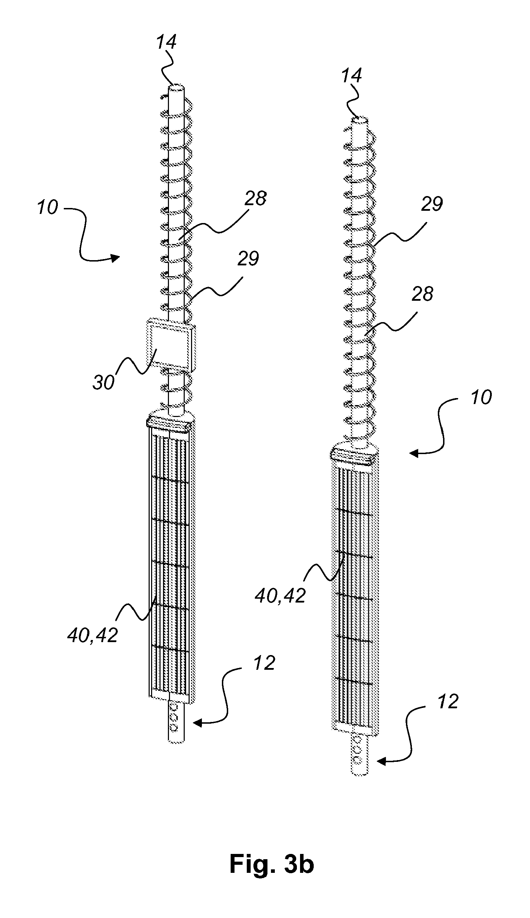

Die schematischen Ansichten der Fig. 3 (a und b) zeigen eine mögliche Ausgestaltung eines Multifunktionselements 10, welches als Stütze zwischen Decke 20 und Boden 24 einer in den Figuren 1 und 2 dargestellten Wärmekabine 1 dient. Dieses säulenartige Multifunktionselement 10 weist eine Zuluftöffnung 12 an einem unteren Ende bzw. in einem Fußbereich des Multifunktionselements 10 auf, um die Luftzufuhr in die Wärmekabine 1 zu ermöglichen. Eine Abluftöffnung 14 befindet sich außerhalb der Decke 20 der Wärmekabine 1, an einem oberen Ende des säulenartigen Multifunktionselements 10. Das Multifunktionselement 10 durchdringt die Decke 20 der Wärmekabine 1 im Bereich einer Deckendurchführung 22; ebenso ist das Multifunktionselement 10 mit dem Boden 24 der Wärmekabine mittels einer Bodendurchführung 26 befestigt. Dieses Multifunktionselement 10 könnte in einer Wärmekabine 1 auch freistehend untergebracht sein. Leitungen 28 für die Luftzufuhr und Luftabfuhr können innerhalb des Multifunktionselements 10, welches als Rohr ausgebildet ist, geführt werden. Eine mögliche Platzierung eines Gehäuses 42 mit wenigstens einem Wärmestrahler 40 ist im vorliegenden Ausführungsbeispiel am unteren Ende des Multifunktionselements 10 angebracht. Die Zuluftöffnung 12 ist allerdings nicht durch das Gehäuse 42 vom Wärmestrahler 40 bedeckt.

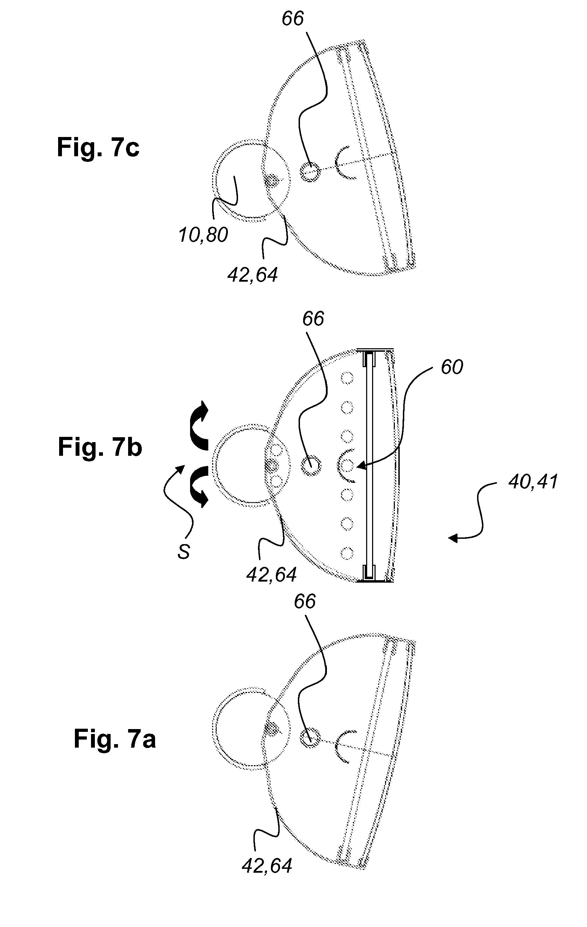

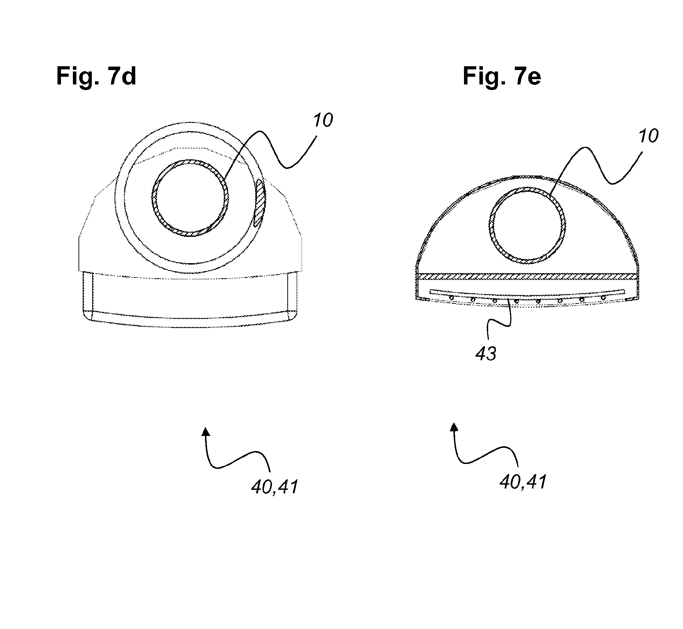

Derartige Wärmestrahler 40 können auch nebeneinander am Multifunktionselement 10 angeordnet sein, bzw. übereinander (nicht dargestellt). Ebenso wäre es denkbar, dass die Wärmestrahler 40 nach allen Seiten schwenkbar (vgl. Fig. 7a) und/ oder verschiebbar angebracht sind, so dass die Wärmestrahler 40 vertikal sowie auch horizontal beweglich angeordnet sind.

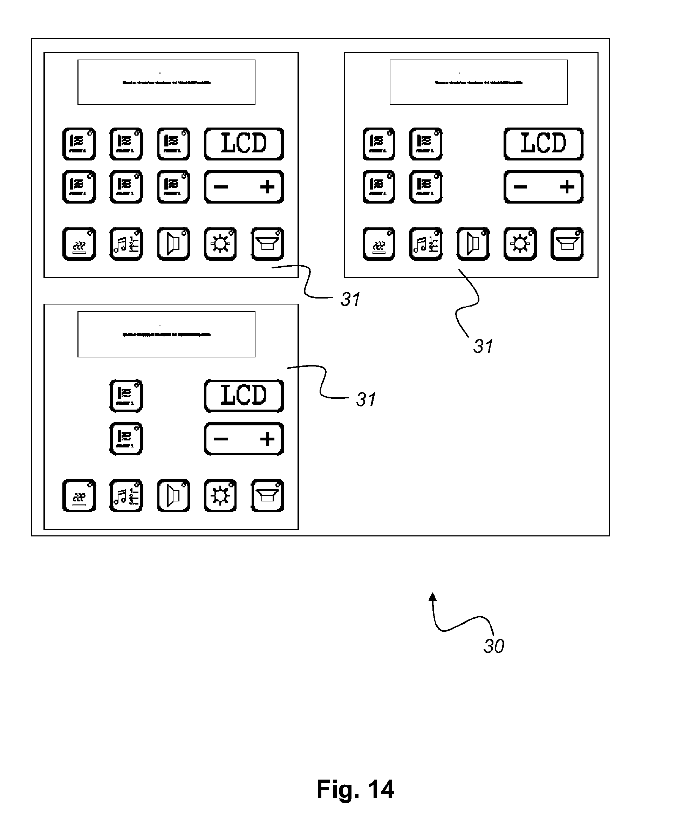

An dem Multifunktionselement 10 ist weiterhin eine Bedieneinheit 30 angeordnet. Diese ist über eine Kabelführung 32 mit einem Lautsprecher 34 über die Rohrleitungen 28 des Multifunktionselements 10 verbunden. Die Bedieneinheit 30 umfasst mindestens ein Schaltelement sowie ggf. Anzeigeeinheiten 31 (vgl. Fig. 14) o. dgl. zur Visualisierung von Betriebs- und/oder Steuerparametern der Komponenten des Multifunktionselements 10, so dass über Benutzereingaben deren Betrieb den Wünschen des Benutzers jeweils angepasst werden kann. Auch wäre es denkbar, dass in diesem Multifunktionselement 10 eine Beleuchtungslampe (nicht dargestellt) integriert ist.

Weiterhin ist in Figur 3b ein Verbrennungsschutz 29 dargestellt. Der Verbrennungsschutz 29 ist um die Rohrleitung 28 des Multifunktionselements 10 gewickelt, damit sich der Anwender keine Verbrennungen durch Berührung der Rohrleitung 28 hinzufügen kann.

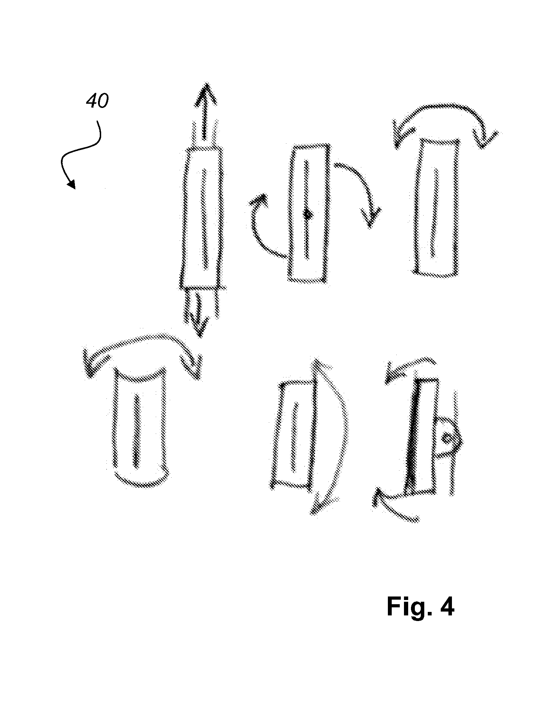

Die Figur 4 zeigt schematische Ansichten von Möglichkeiten zur Positionsveränderung von Wärmestrahlern 40 mit Infrarotstrahlern 41 der beanspruchten Wärmekabine 1. Die Infrarotstrahler 41 können stufenlos höhenverstellbar als Front-, Seiten- und Rückenstrahler ausgebildet sein. Des Weiteren können diese Infrarotstrahler 41 in der Wärmekabine 1 stufenlos drehbar und/ oder in alle Richtungen stufenlos schwenkbar angebracht sein. Diese Bewegungsmöglichkeiten sind mit Pfeilen dargestellt.

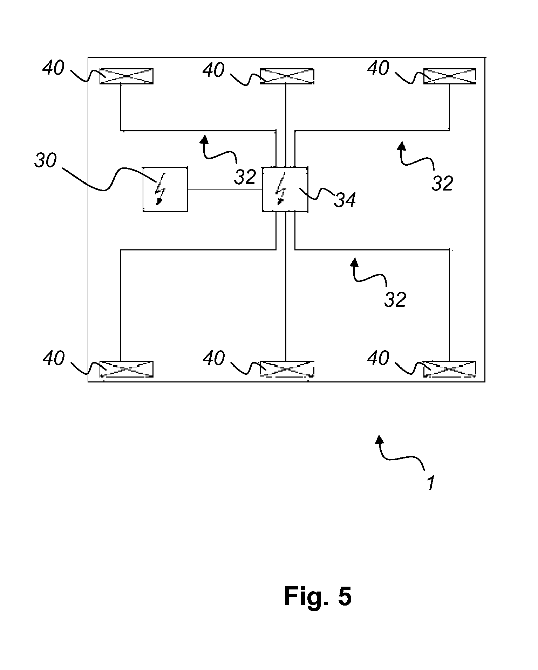

Die schematische Darstellung der Fig. 5 zeigt eine Draufsicht einer möglichen Anordnung von Wärmestrahlern 40 in einer Wärmekabine 1. Die einzelnen Wärmestrahler 40 sind über Kabelführungen 32 mit der Bedieneinheit 30 über ein Leistungsschaltgerät 34 verbunden und können somit einzeln als auch zusammen über ein Programm angesteuert werden. In der Fig. 5 ist eine beispielhafte Anordnung von insgesamt sechs Wärmestrahlern 40 gezeigt. Es sind jedoch auch mehr oder weniger Wärmestrahler 40 in einer Wärmekabine 1 denkbar (nicht dargestellt, da es sich um eine ähnliche Anordnung handelt). Die Bedieneinheit 30 kann an dem Multifunktionselement 10 (vgl. Fig. 3) angebracht sein.

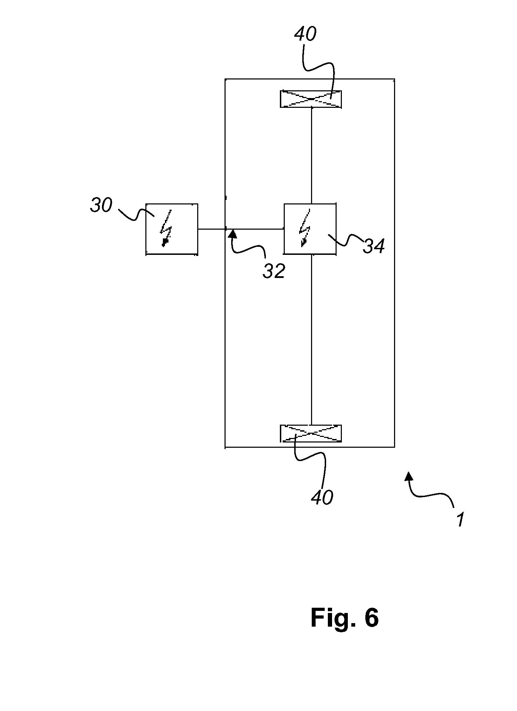

Die schematische Darstellung der Fig. 6 zeigt eine weitere mögliche Anordnung von bspw. zwei Wärmestrahlern 40 in einer Wärmekabine 1. Bei dieser Ausführungsform ist die Bedieneinheit 30 außerhalb der Wärmekabine 1 angeordnet und mit einem Leistungsschaltgerät 34 über eine Kabelführung 32 mit den Wärmestrahlern 40 verbunden. Diese Anordnung der Bedieneinheit 30 außerhalb der Wärmekabine 1 wäre auch bei der in Fig. 5 dargestellten Ausführungsform möglich.

Die Figuren 7a bis 7c zeigen Querschnitte von Wärmestrahlern 40 in unterschiedlichen Positionen. Die Infrarotstrahler 41 der Wärmestrahler 40 weisen Primärreflektoren 60 sowie mindestens eine Infrarotlampe 66 auf. Diese sind in einem Gehäuse 42, welches nach einer Seite offen ist, eingebracht. Damit von der Infrarotlampe 66 eine gleichmäßige und flächenhafte gestreute Strahlung durch die Öffnung des Gehäuses 42 in eine gewünschte Richtung austritt, sind die Infrarotstrahler 41 mit ihren Primärreflektoren 60 innerhalb des Gehäuses 42 derart ausgebildet, dass die ausgesandte Strahlung ausschließlich auf eine Innenwandung 64 des Gehäuses 42 trifft.

Aus der Figurenfolge 7a bis 7c ist ersichtlich, dass die Wärmestrahler 40 beweglich am Multifunktionselement 10 oder an einer ähnlichen Halterung 80, welche bspw. als Rohr ausgebildet ist, angebracht sein können. Die Schwenkbewegung S der Wärmestrahler 40 kann in horizontaler oder vertikaler Lage der Wärmestrahler 40 erfolgen oder nach oben und unten dementsprechend wie der Strahler 40 angebracht ist.

In der Figurenfolge 7d bis 7e ist ersichtlich, dass die Wärmestrahler 40 unbeweglich oder um ihre Längsachse verdrehbar und/oder höhenverstellbar am -ggf. ebenfalls um seine Achse verdrehbaren - Multifunktionselement 10 angebracht sind und ein Schutzgitter 43 umfassen. Dieses Schutzgitter 43 dient dazu, dass sich der Anwender keine Verbrennungen durch die Infrarotlampen 66 (vgl. Fig. 7a - c) hinzufügen kann.

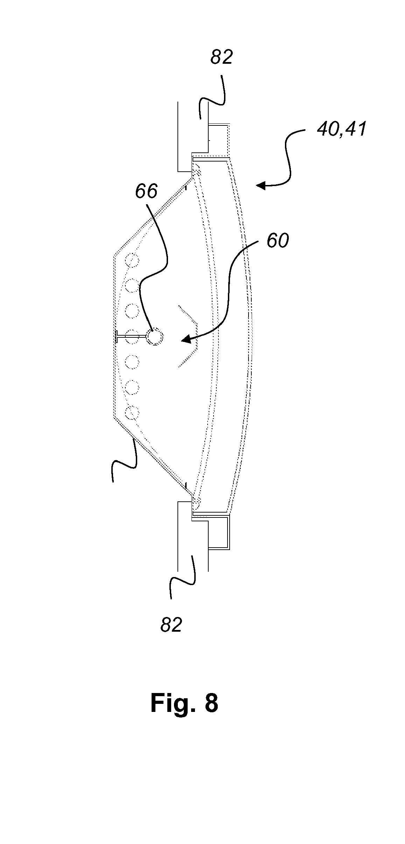

Der Wärmestrahler 40 aus der Figur 8 ist bspw. nicht schwenkbar gelagert, sondern verschiebbar. Dieser Wärmestrahler 40 wird in Schienen 82 eingeklemmt und kann somit bspw. nach oben und unten bewegt werden. Im gezeigten Ausführungsbeispiel entspricht die Bewegung des Wärmestrahlers 40 aus der Blattebene heraus und in die Blattebene hinein. Auch ist in dieser Figur 8 ein Reflektor 60 eingebaut, über den die Strahlung von der Infrarotlampe 66 auf die Innenwandung 64 zurückgelenkt werden kann.

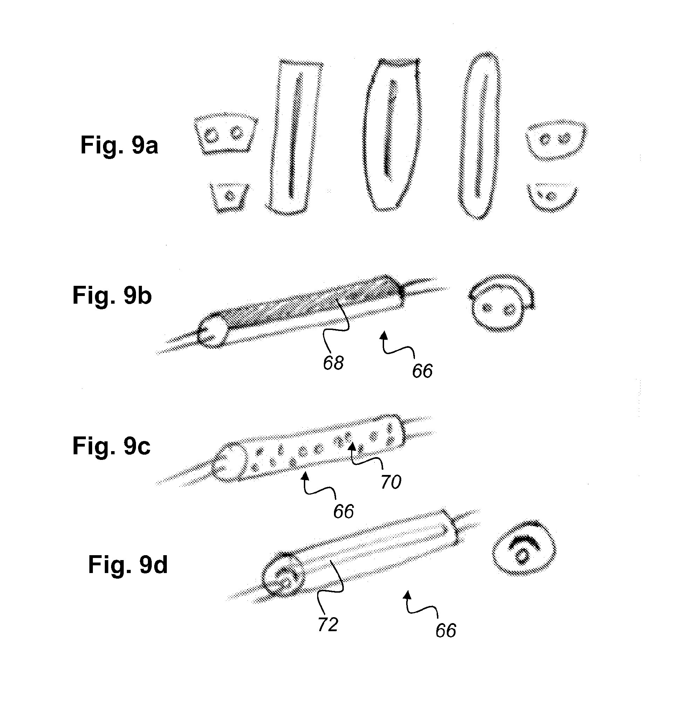

Aus den Figuren 9a bis 9d werden einige mögliche Ausführungsformen einer Infrarotlampe 66 dargestellt, wie diese als Bestandteil eines Wärmestrahlers 40 ausgestaltet werden können. Aus der Figur 9a sind bspw. unterschiedlichen Formen und Schnitte von unterschiedlichen Gläsern abgebildet, die für Infrarotstrahler 41 Verwendung finden können. In der Figur 9b ist die Infrarotlampe 66 komplett beschichtet, jedoch ist auch eine teilweise Beschichtung 68 der Infrarotlampe 66 möglich. Infrarotlampen 66 nach dieser Figur 9b erzielen ein optimales Strahlungsspektrum und/ oder einen optimalen Austrittswinkel der Infrarotstrahlung. Die in Abbildung 9c verwendete Infrarotlampe 66 wird mit einer Mischung aus Quarz- und Lavasand betrieben. Diese Mischung dient zur Erzielung einer wirksamen und lang anhaltenden Infrarotstrahlung, die bereits unmittelbar nach der Abschaltung der Infrarotlampe 66 eintritt. Eine Infrarotlampen 66 mit einem eingebauten Konterreflektor 72 ist aus der Figur 9d ersichtlich. Eine derartige Infrarotlampe 66 mit eingebauten Konterreflektor 72 dient zur Erzielung eines optimalen Strahlungsspektrums und/ oder eines optimalen Austrittswinkels der Infrarotstrahlung.

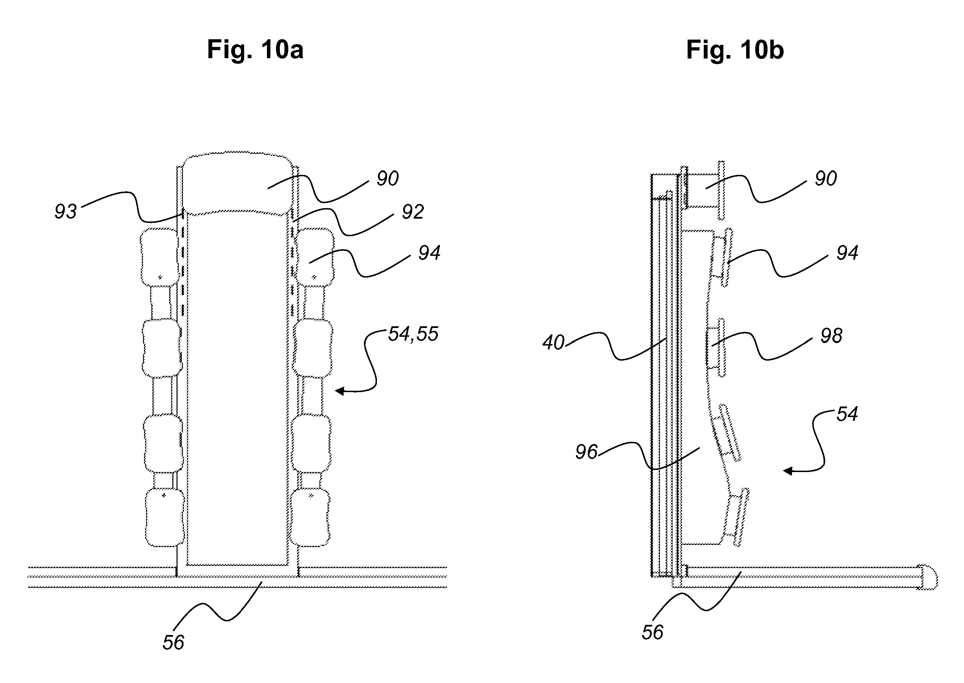

Des Weiteren zeigen die Figuren 10a und 10b schematische Ansichten einer möglichen Anordnung eines Rückenelements 54. Die Rückenelemente 54 sind ergonomisch geformt, so dass sich diese besser an die Rückenkontur des Menschen anpassen können. Die Rückenelemente 54 sind in getrennte Lehnenabschnitte 55 angeordnet, zwischen diesen ein Wärmestrahler 40 (vgl. Fig. 10b) angebracht sein kann. Der Wärmestrahler 40 sorgt für eine optimale und hindernisfreie Bestrahlung des gesamten Rückens im Wirbelsäulenbereich und durch Abstandshalter 98 wird ein Kontakt des Rückens mit dem Wärmestrahler 40 vermieden (vgl. Fig. 10b) und somit sinkt die Verletzungsgefahr. Die beiden Lehnenabschnitte 55 sind an die Rückenkontur des Menschen angepasst, d. h. die beiden Lehnenabschnitte 55 laufen am unteren Ende zum Sitzelement 56 aufeinander zu und nach oben voneinander weg. Durch diese Anordnung bildet sich eine V-Anordnung, die dem menschlichen Rücken gleicht. Am oberen Ende der beiden Lehnenabschnitte 55 kann eine Kopfstütze 90 angebracht sein. Diese Kopfstütze 90 entspricht ebenfalls der Kopfform des Menschen, so dass die Oberfläche leicht nach außen gewölbt ist. Zudem lässt sich diese Kopfstütze 90 anhand eines Einrastsystems 92 in der Höhe verstellen. Dieses Einrastsystem 92 ist hinter den Rückenelementen 54 angeordnet. Die Kopfstütze 90 wird mit Haken (nicht dargestellt) in entsprechende Lochstreifen 93 des Einrastsystems 92 eingehängt und somit befestigt.

Die Figur 10b zeigt eine schematische Seitenansicht einer möglichen Ausführungsform eines Rückenelementes 54. Das Rückenelement 54 kann eine unterschiedliche Anzahl von Lehnensegmenten 94 aufweisen. Im vorliegenden Ausführungsbeispiel sind vier Lehnensegmente 94 von gleicher Große an einem Rückenlehenbefestigung 96 angebracht, jedoch ist es durchaus möglich verschiedene große Lehnensegmente 94 zu verwenden. Die Lehnensegmente 94 haben vorzugsweise eine rechteckige Form (vgl. Fig. 10a) und eine flache und/ oder leicht nach außen gewölbte Oberfläche. Diese Lehnensegmente 94 sind an den Abstandshaltern 98 fest angebracht.

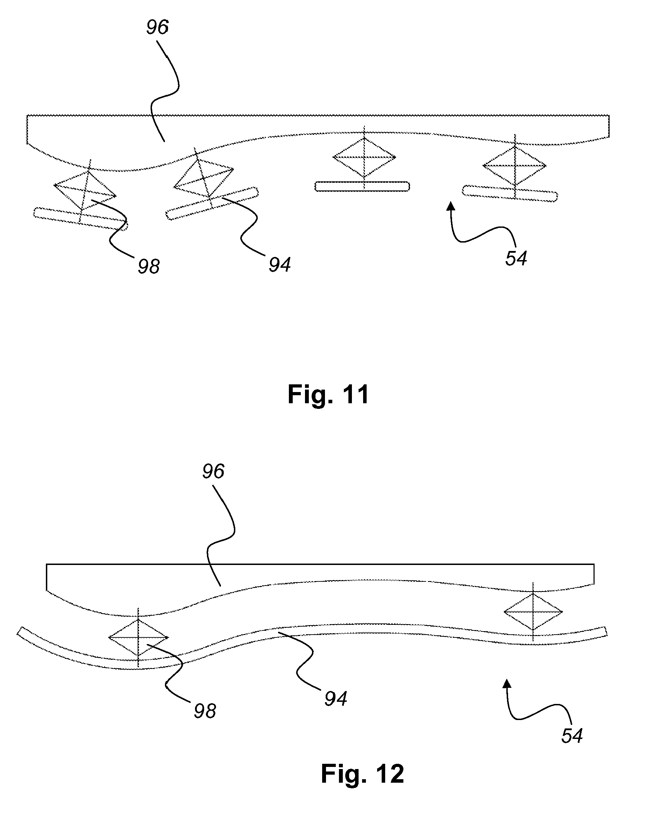

Eine weitere Ausführungsform eines Rückenelements 54 wird in der Figur 11 gezeigt, die eine schematische Seitenansicht darstellt. Bei dieser Figur sind die Lehnensegmente 94 jedoch flexibel ausgestattet. Die Flexibilität wird durch die federnden und gelenkig gelagerten Lehnensegmente 94 ermöglicht. Die Abstandshalterungen 98 sind unterhalb der Lehnensegmente 94 angeordnet und zudem an der Rückenlehenbefestigung 96 befestigt. Diese Lehnensegmente 94 ermöglichen durch die flexible Lagerung an den Abstandshalterung 98 eine bessere Anpassungsfähigkeit an den Rücken des Anwenders.

Die Figur 12 zeigt eine schematische Seitenansicht einer weiteren möglichen Ausführungsform eines Liege- oder Sitzelementes, das integraler Bestandteil einer beanspruchten Wärmekabine 1 sein kann. Bei dieser Ausführungsform ist das Lehnensegment 94 als ganzes Teil ausgebildet und nicht wie in Figur 11 als einzelne Lehnensegmente 94. Das Lehnensegment 94 ist ebenso der Rückenform angepasst. Die Rückenlehenbefestigung 96 ist ähnlich geformt wie bei den Ausführungsbeispielen davor und an diesen Abschnitten werden die Abstandhalterungen 98 angebracht, um das Lehnensegment 94 befestigen zu können und um die Flexibilität dessen herzustellen.

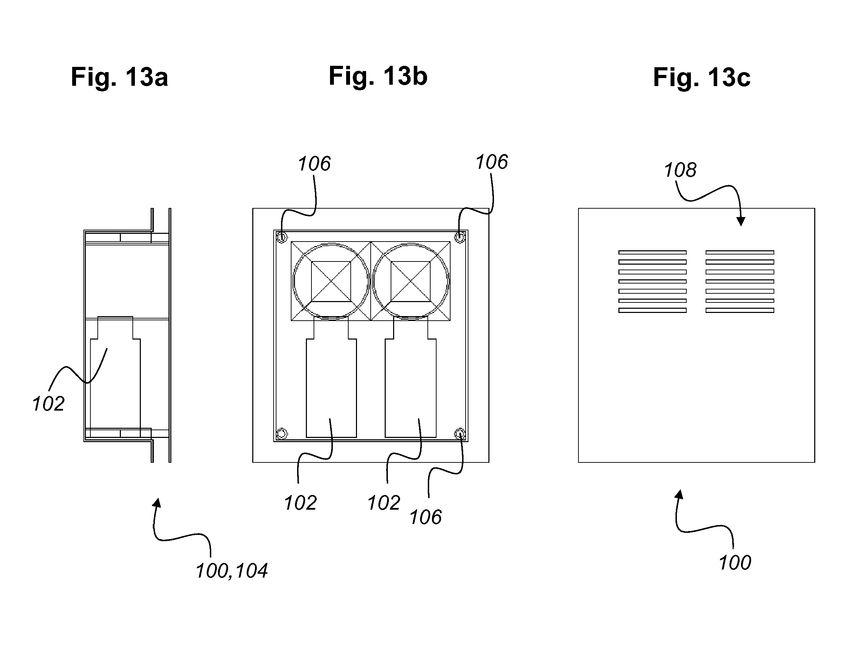

Die Figur 13a bis 13c zeigen verschiedene Ansichten eines Duftzerstäubers 100, wie dieser in einer beanspruchten Wärmekabine 1 vorhanden sein kann. Aus der Figur 13a ist eine Seitenansicht, aus der Figur 13b eine Rückansicht und aus der Figur 13c eine Vorderansicht des Duftzerstäubers 100 ersichtlicht. Dieser Duftzerstäuber 100 ist eine weitere vorteilhafte Ausführungsvariante eines Duftspenders oder Zerstäubers oder dgl. Ein Duftölbehälter 102 wird in eine Vorrichtung 104 eingesetzt. Diese Vorrichtung 104 besteht vorzugsweise aus gleichem Material wie die Wärmekabine 1 und wird in der Wärmekabine 1 bspw. an die Kabinenrückwand 44 im oberen Bereich zur Decken 20 befestigt. Für die Befestigung des Duftzerstäubers 100 sind Löcher 106 an der Vorrichtung 104 vorgesehen (vgl. Fig. 13b), damit der Duftzerstäuber 100 mit der Rückwand 44 verschraubt werden kann.

Die Bedienung des Duftzerstäubers 100 erfolgt bspw. mittels eines Ventilators (nicht dargestellt). Dieser Ventilator kann den Duftzerstäuber 100 mit Frischluft von außen beaufschlagen, so dass an dem Duftspender 100 die vorbei geführte Frischluft mit einer definierbaren Menge eines Geruchs- oder Duftträgers beaufschlagt und anschließend über mehrer Belüftungsschlitze 108 in die Wärmekabine 1 geleitet werden kann. Je nach Ansteuerung (nicht dargestellt) des Duftspenders 100 bzw. Zerstäubers kann die Menge an in die Kabine 1 geleitetem Geruchs- oder Duftträger reguliert werden.

Das erwähnte Beduftungssystem kann wahlweise als integriertes Bestandteil des Multifunktionselements 10 (vgl. Fig. 1) bzw. eines der Multifunktionselemente sein oder ein separates Element darstellen, das zweckmäßigerweise in die Rückwand 44 oder in ein Deckenelement 20 der Wärmekabine 1 eingebaut werden kann. Das Beduftungssystem ist im Zusammenhang mit einer Frischluftzuführung (nicht dargestellt) zu sehen.

Die Figur 14 zeigt mögliche Ausführungsformen zur Ausgestaltung von Bedieneinheiten 30, durch die der Betrieb einer beanspruchten Wärmekabine 1 geregelt werden können. Durch die Bedieneinheit 30 sind umfassende Zugriffsmöglichkeit auf Musik- und/oder Bildmedien, die auf geeigneten Speicherinhalten abgelegt sind, möglich. So kann die Bedieneinheit 30 z.B. zur Musikwiedergabe ausgerüstet sein, wahlweise zum Datenempfang von einer Steuerungseinheit und/oder zur Wiedergabe aus einem eigenen Speicher und/oder Datenträger. So kann bspw. in der Bedieneinheit 30 ein MP3-Spieler (nicht dargestellt) o. dgl. integriert sein. Zudem können in der Bedieneinheit 30 und/ oder in der dieser zugeordneten Multifunktionselement 10 (vgl. Figur 1) Lautsprecher 36 (vgl. Figur 1) integriert sein, wobei das Multifunktionselement 10 ggf. als Klangkörper genutzt werden kann.

Für die Betätigung der verschiedenen Funktionen mittels der Bedieneinheit 30 sind bereits zahlreiche Prinzipien bekannt. So eignen sich bspw. berührungsempfindliche Bildschirme zur Eingabe von Steuerbefehlen. Daneben können Folientastaturen oder berührungsempfindliche Bedienfelder zur Benutzereingabe dienen. Derartige berührungsempfindliche Felder oder Bildschirme weisen den Vorteil auf, dass eine gegen Schmutz unempfindliche und leicht gegen Feuchtigkeitseinflüsse abzuschirmende Eingabetastatur zur Verfügung gestellt wird. Diese Eingabetastatur dient als Anzeigeeinheit 31, so dass diese je nach Bedarf grafisch gestaltet werden können, um dem Benutzer die Betätigung zu erleichtern.

Die in den Figuren beschriebenen Ausführungsvarianten zeigen lediglich beispielhafte Konfigurationen und Anordnungen von Wärmestrahlern und deren Ausrichtung innerhalb der Kabine. Das dargestellte Bedienteil muss nicht zwingend mit dem Multifunktionselement verbunden sein, sondern kann mit dessen Komponenten ggf. auch über eine drahtlose Datenverbindung kommunizieren, so dass das Bedienteil ggf. abnehmbar und/oder völlig von der Säule des Multifunktionselements trennbar ausgeführt sein kann. Auch kann das Bedienteil wahlweise zur Steuerung weiterer Komponenten wie bspw. der Liegen und/oder der steuerbaren Magnetfelder sowie ggf. weiterer, hier nicht näher erwähnter Parameter dienen.

Es ist darüber hinaus für den Fachmann selbstverständlich, dass auch andere Konfigurationen als hier beschrieben möglich sind, bspw. der Einbau von zwei oder mehr derartiger Multifunktionselemente in größeren Wärmekabinen, deren Komponenten vorzugsweise in sinnvoller Abstimmung miteinander gemeinsam oder getrennt ansteuerbar sind, um ein möglichst günstiges Klima und möglichst günstige Bedingungen für die Insassen der Wärmekabine schaffen zu können.

### Bezugszeichenliste

- 1: Wärmekabine, Kabine
- 10: Multifunktionselement
- 12: Zuluftöffnung
- 14: Abluftöffnung
- 20: Decke
- 22: Deckendurchführung
- 24: Boden, Kabinenboden
- 26: Bodendurchführung
- 28: Rohrleitung, Leitung
- 29: Verbrennungsschutz
- 30: Bedieneinheit
- 31: Anzeigeeinheit
- 32: Datenleitung/Kabelführung
- 34: Leistungsschaltgerät
- 36: Lautsprecher
- 38: Beleuchtungseinrichtung
- 40: Wärmestrahler
- 41: Infrarotstrahler
- 42: Gehäuse
- 43: Schutzgitter
- 44: Rückwand
- 46: Seitenwand
- 48: Vorderwand
- 50: Wanne, Wannenboden
- 52: Liegeelement
- 54: Rückenelement, Rückenlehne
- 55: Lehnenabschnitt
- 56: Sitzelement, Sitzbank
- 60: Primärreflektoren
- 62: gebogenes Roblaxglas
- 64: Innenwandung
- 66: Infrarotlampe

- 68: Beschichtung
- 70: Quarz- und Lavasand
- 72: Konterreflektor
- 80: Halterung
- 82: Schienen
- 90: Kopfstütze
- 92: Einrastsystem
- 93: Lochstreifen
- 94: Lehnensegment
- 96: Rückenlehenbefestigung
- 98: Abstandshalterung
- 100: Duftzerstäuber
- 102: Duftölbehälter
- 104: Vorrichtung
- 106: Löcher
- 108: Belüftungsschlitze
- S: Schwenkbewegung

## Patentansprüche

1. Wärme- und/oder Infrarotkabine (1) für mindestens eine Person, mit einem Boden (24) und einer Decke (20) und sich einer zwischen dem Boden (24) und der Decke (20) erstreckenden umlaufenden Seitenwand (46), mit mindestens einem säulenartigen, länglichen und/oder drehbar gelagertes Multifunktionselement (10) und/oder -bauteil, das mit dem Boden (24) und/oder der Decke (20) und/oder der Seitenwand (46) verbunden ist, wobei das Multifunktionselement wenigstens eine, insbesondere jedoch mehrere der folgenden Elemente aufweist bzw. Funktionen erfüllt (10):
- eine statische und/oder tragende Funktion, insbesondere zur Aufhängung und/oder zur Stützung weiterer Funktionselemente;
- Anordnung wenigstens eines Wärmestrahlers am oder im Multifunktionselement;
- Anordnung wenigstens einer Ausströmstelle für Aroma- und/oder Duftstoffe;
- Luftführung, -leitung, insbesondere mittels innerhalb des Multifunktionselements (10) geführter bzw. verlaufender Kanäle und/oder Leitungen und/oder Belüftungsfunktion, insbesondere mittels innerhalb und/oder außerhalb der Kabine (1) angeordneter Ein- und Ausströmöffnungen;
- Anordnung wenigstens einer Steuerungs-, Bedienungs- und/oder Anzeigeeinheit;
- Führung von Versorgungsleitungen, bspw. für Luft, Wasser, Aromastoffe etc. innerhalb von Kanälen im Multifunktionselement;
- weitere Funktionen wie Handtuchhalter etc., insbesondere durch variable Form, variablen Verlauf und Gestaltung.

2. Kabine nach Anspruch 1, bei der das Multifunktionselement (10) als ein statisches und/oder tragendes und/oder konstruktives und/oder funktionales Bauteil der Wärme- bzw. Infrarotkabine (1) und/oder als Rohr mit wenigstens zwei Kammern und/oder mit Kabelführungen (32) ausgebildet ist.

3. Kabine nach Anspruch 1 oder 2, bei der dem Multifunktionselement (10) und/oder der Wärmekabine (1) mindestens ein Infrarot- und/oder Wärmestrahler (40) zugeordnet ist, der integraler Bestandteil des Multifunktionselementes (10) oder an diesem befestigt ist und/oder der an der Seitenwand (46) angeordnet ist.

4. Kabine nach Anspruch 3, bei der wenigstens ein am Multifunktionselement (10) angeordneter oder diesem zugeordneter Wärmestrahler (40) horizontal nach allen Seiten schwenkbar und/oder vertikal neigbar und/oder entlang einer Längserstreckungsrichtung des Multifunktionselements (10) verschiebbar ausgebildet ist und/oder dass das Multifunktionselement selbst drehbar gelagert ist.

5. Kabine nach einem der Ansprüche 1 bis 4, bei der die Öffnungsquerschnitte der Zu- und/oder Abluftöffnungen (12,14) variabel steuerbar und/oder verschließbar sind.

6. Kabine nach einem der Ansprüche 1 bis 5, bei der zumindest in einer Seitenwand (46) und/oder der Decke (20) der Wärmekabine (1) und/oder am bzw. im Multifunktionselement (10) wenigstens ein Aromasystem und/oder ein Duftzerstäuber (100) angeordnet ist.

7. Kabine nach einem der Ansprüche 1 bis 6, bei der eine Bedien- und/oder Anzeigeeinheit (30) fest oder lösbar mit dem Multifunktionselement (10) verbunden ist, über die dem Multifunktionselement (10) und/oder der Wärmekabine (1) zugeordnete Funktionen und/oder Parameter einstellbar und/oder veränderbar sind.

8. Kabine nach einem der Ansprüche 1 bis 7, bei der dem Multifunktionselement (10) Einrichtungen zur Akustikwiedergabe, insbesondere zur Medienwiedergabe, zur akustischen Übertragung von Ansagen, von Musik, von Hinweisen etc. und/oder zur visuellen Wiedergabe, insbesondere von Videos o. dgl. zugeordnet sind.

9. Kabine nach einem der Ansprüche 1 bis 8, bei dem das Multifunktionselement (10) als Leuchtkörper ausgebildet ist und flächige und/oder punktuelle Lichtaustrittsstellen aufweist.

10. Kabine nach einem der Ansprüche 1 bis 9, bei der eine dem Multifunktionselement (10) zugeordnete oder an diesem angeordnete Bedieneinheit (30) mit einer Steuerungseinheit verbunden ist, die mit jedem einzelnen Wärmestrahler (40) in der Wärmekabine (1) kommunikativ verbunden ist, so dass die Wärmeleistung jedes einzelnen Infrarot- oder Wärmestrahlers (40) selektiv regelbar ist.

11. Kabine nach einem der Ansprüche 1 bis 10, bei der zumindest einer der Strahler (40), insbesondere alle Strahler (40) eine kaminartige Hinterlüftung zur Kühlung des Strahlers (40) aufweist/aufweisen.

12. Kabine nach einem der Ansprüche 1 bis 11, bei dem das Multifunktionselement (10) einen passiven Verbrennungsschutz aufweist, insbesondere in Form einer Oberflächenbeschichtung, und/oder mit wenigstens einem wärmeisolierten Griff versehen ist, zum Schwenken, Schieben, Drehen, Wenden etc. des wenigstens einen Strahlers (40) und/oder des Multifunktionselements (10).

13. Kabine nach einem der Ansprüche 1 bis 12, bei dem das Multifunktionselement (10) einen aktiven Verbrennungsschutz aufweist, insbesondere in Form einer Sensoreinrichtung zur Messung der Hauttemperatur eines Benutzers und einer damit gekoppelten selektiven Steuerung der einzelnen Strahler (40), wobei eine Sitzbelegungserkennung bei nicht vom Sensor erfassbarer Hauttemperatur den zugehörigen Strahler (40) deaktiviert.

14. Kabine nach einem der Ansprüche 1 bis 13, bei der wenigstens ein Sitzbereich (56) eine ergonomisch geformte Rückenlehne (54) aufweist, wobei die Rückenlehne (54) wenigstens zwei im Wesentlichen vertikal verlaufende, beabstandet voneinander angeordnete Lehnenabschnitte (55) aufweist und/oder bei der der Rückenlehne (54) wenigstens ein Wärmestrahler (40) zugeordnet ist, dessen Abstrahlbereich auf den Rücken der den Sitzbereich (56) benutzenden Person gerichtet ist, und/oder bei der die Rückenlehne (54) und/oder die wenigstens zwei Lehnenabschnitte (55) in mehrere, vertikal voneinander beabstandete Lehnensegmente (94) unterteilt sind, von denen zumindest einige, vorzugsweise jedoch alle, flexibel und/oder federnd aufgehängt und/oder an der Seitenwand (46) oder Rückwand (44) der Wärmekabine (1) aufgehängt sind.

15. Kabine nach einem der Ansprüche 1 bis 14, bei der das Multifunktionselement (10) mit leitungsführenden Kanälen, Schächten etc. ausgebildet und insbesondere hohl, mit innenliegendem Hohlraum versehen ist, der in Längsrichtung mindestens einmal unterteilt ist zur Bildung von zwei oder mehr Schächten zur Aufnahme von Versorgungsleitungen aller Art wie bspw. Elektrokabel, Glasfaser, Luftschläuche etc., Aromaleitungen, Duftleitungen etc.
